(19) **Europäisches Patentamt / European Patent Office / Office européen des brevets**

(11) **EP 4 635 259 B1**

(12) **EUROPEAN PATENT SPECIFICATION**

(45) Date of publication and mention
of the grant of the patent:
**22.07.2026 Bulletin 2026/30**

(21) Application number: **23817167.2**

(22) Date of filing: **05.12.2023**

(51) International Patent Classification (IPC):
*H05B 45/20* (2020.01)   *A61N 5/06* (2006.01)
*H10H 20/856* (2025.01)   *H10W 90/00* (2026.01)

(52) Cooperative Patent Classification (CPC):
**H05B 45/20; A61N 5/0618;** H10H 20/856;
H10W 90/00

(86) International application number:
**PCT/EP2023/084212**

(87) International publication number:
**WO 2024/126148 (20.06.2024 Gazette 2024/25)**

(54) **CYAN ENHANCED SOURCES WITH 2° MATCHED LEDS AND CORRECT 10° COLOR POINTS**

CYANVERSTÄRKTE QUELLEN MIT 2 2 ADAPTIERTEN LEDS UND KORREKTEN 10-FARBPUNKTEN

SOURCES CYAN AMÉLIORÉES AVEC DES DEL ADAPTÉES À 2 ET POINTS DE COULEUR CORRECTS DE 10

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **15.12.2022 EP 22213823**

(43) Date of publication of application:
**22.10.2025 Bulletin 2025/43**

(73) Proprietor: **Signify Holding B.V.
5656 AE Eindhoven (NL)**

(72) Inventors:
• **PEETERS, Martinus, Petrus, Joseph**
**5656 AE Eindhoven (NL)**
• **WEGH, René, Theodorus**
**5656 AE Eindhoven (NL)**

(74) Representative: **Verweij, Petronella Daniëlle
Signify Netherlands B.V.
Intellectual Property
High Tech Campus 7
5656 AE Eindhoven (NL)**

(56) References cited:
**US-A1- 2018 056 027     US-A1- 2021 329 757**

**Description**

FIELD OF THE INVENTION

The invention relates to a light generating system as well as to a lamp or luminaire comprising such light generating system.

BACKGROUND OF THE INVENTION

Solid state light emitting devices including adjustable melatonin suppressing effects are known in the art. US 9,039,746, for instance, describes a solid state light emitting device including multiple LED components providing adjustable melatonin suppression effects. Multiple LED components may be operated simultaneously according to different operating modes according to which their combined output provides the same or similar chromaticity, but provides melatonin suppressing effects that differ by at least a predetermined threshold amount between the different operating modes. Switching between operating modes may be triggered by user input elements, timers/clocks, or sensors (e.g., photo sensors). Chromaticity of combined output of multiple LED components may also be adjusted, together with providing adjustable melatonin suppression effects at each selected combined output chromaticity.
US 2018/0056027 A1 discloses a three-channel lighting apparatus with the option to support the human circadian rhythm.

SUMMARY OF THE INVENTION

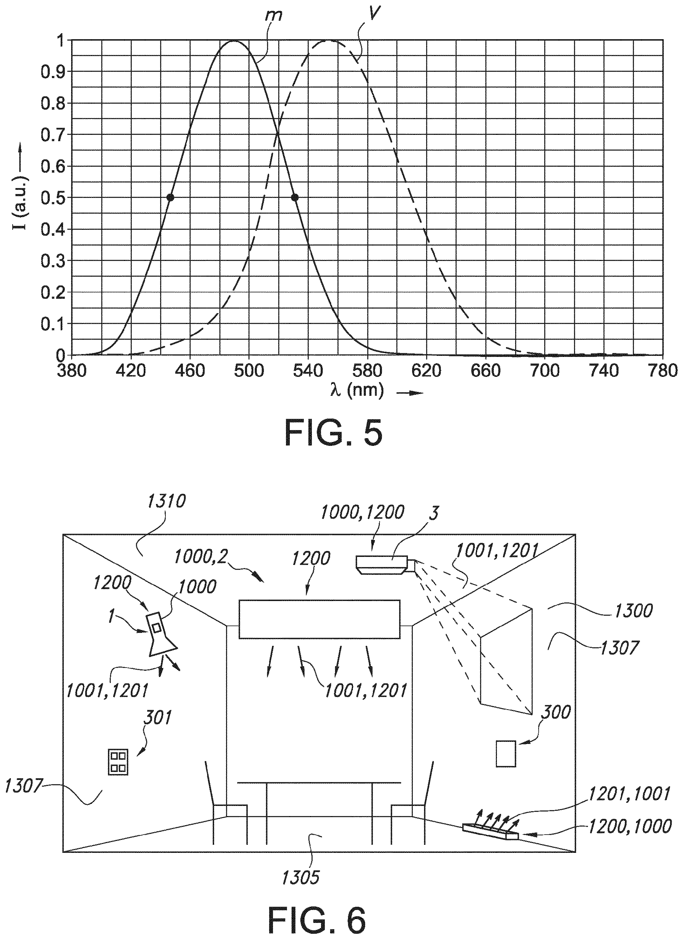

Critical to our sleep/wake cycle is melatonin, a hormone that promotes sleep during nighttime. Melatonin is a sleep supportive hormone that we only produce around (and during) our usual bedtime. Light exposure during the evening and at night suppresses the natural production of melatonin. When the spectrum of the light is shifted towards lower CCT and intensity levels (like during dawn and dusk), this reduces melatonin suppression and makes the light less disruptive for sleep. During daytime, natural daylight with high correlated color temperature (CCT, herein also indicated as "color temperature") and intensity energizes people making them awake and alert. Current high performance LED based lighting apparatus with tunable CCT are able to mimic different phases of daylight, i.e., changes in spectral power distribution and variations in CCT, to a certain extent.
Next to the commonly known cones and rods, the human eye has melanopsin containing photoreceptors, affecting circadian entrainment and melatonin secretion, which are sensitive in a specific wavelength range. The relative spectral sensitivity for the classic receptors (rods and cones) and for the melanopic receptors are provided in Fig. 5 (see also R.J. Lucas, et al., Measuring and using light in the melanopsin age, Trends in Neurosciences, Vol. 37, No. 1, January 2014, pp. 1-9; http://www.sciencedirect.com/science/article/pii/S0166223613001975, the report "CIE TN 003:2015 : Report on the First International Workshop on Circadian and Neurophysiological Photometry, 2013" at http://cie.co.at/index.php?i_ca_id=978 (with a link to an excel toolbox http://files.cie.co.at/784_TN003_Toolbox.xls).
If the spectral power in the melanopic wavelength range is absent or low, the light exposure will be less suppressive for the melatonin hormone production thus enabling faster sleep onset and more consolidated sleep. If the spectral power in the melanopic range is increased, a light exposure will result in stronger melatonin suppression. In general a light exposure can be said to be more biologically active and more alerting when the power in the melanopic range (and the ability to suppress melatonin at night) is increased.
The effectiveness of a given light spectrum in suppressing melatonin production can be expressed in terms of the melanopic daylight efficacy ratio (MDER). The melanopic efficiency of a light spectrum can be calculated using the MDER, i.e. Melanopic Daylight Efficacy Ratio). In such instance, instead of an equal energy light source, a D65 source, i.e. CIE Standard Illuminant D65, which is a commonly used standard illuminant defined by the International Commission on Illumination (CIE), is used as reference. MDER can be defined as the illuminance in lux of a D65 source needed to generate the same stimulation of the iPRGCs per lux of the test source (or test system). The term MELR refers to melanopic efficacy of luminous radiation (in mW/Lm).
Instead of the MDER value, also a MELR value (Melanopic efficacy of luminous radiation) may be used. With respect to the calculation of the MDER value and the MELR value the following can be mentioned. For the test spectrum that is to be evaluated one may calculate how many mW are in the iPRGC-sensitive region of the spectrum of the test source (by weighing the spectrum with m(□). One can also calculate how many Lm are generated. The ratio of melanopic power in mW and lumen in Lm is called MELR value. For a D65 reference spectrum this calculation can also be done. The MELR of D65 = 1.326 mW/Lm. The ratio of the MELR value of the test spectrum to be evaluated and the MELR value of the reference spectrum (D65) is called MDER. MDER is a value without units.

MELR can thus be expressed in mW/Lm in which the mW is calculated by $\sum_{\lambda=380}^{780} \text{SPD}(\lambda)\text{m}(\lambda)\Delta\lambda$. The lumens

in Lm are calculated in the normal way. Herein, SPD(□) is the spectral power distribution of the light emitted by a light generating device, m(□) is the melanopic sensitivity function, the V(□) is the photopic luminosity function.

As indicated above, especially the MDEF, which is herein further indicated as MDER, is applied. The MDER is defined as:

$$melanopic - DER = \frac{melanopic - ELR \ of \ test \ source}{melanopic - ELR \ of \ D65 \ source} = \frac{melanopic - ELR \ of \ test \ source}{1.326}$$

As indicated above, the biological effect of lighting is the product of Illumination (Lux at the eye) * MDER * (Exposure time). Next to that also the time of exposure (morning/evening) determines the effect on people. In normal indoor lighting conditions, the stimulation of the IPRGCs during daytime is too low (e.g. 500 lux on the desk in offices, 4000 K, MDER ~ 0.6).

It appears desirable to enrich lighting with blueish-cyan like light. Hence, high melanopic lighting uses blueish-cyan like light enriched spectra. To be able to choose or tune the MDER value of the light, it appears desirable to use two different types of white light sources, i.e. a light source having a relatively high relative spectral power in the 470-500 nm wavelength range and a light source having a lower relative spectral power in the 470-500 nm range, but a relatively higher spectral power in the 440-470 nm range. The latter light may have a relatively lower MDER value. The former may have relatively more spectral power in the longer blue wavelength range and/or cyan wavelength range, and the latter may have relatively more spectral power in the blue and shorter blue wavelength range. Even though having essentially the same color points (using 2° color matching functions), and having essentially the same correlated color temperature, the two types of light may have substantially different MDER values (such as about a factor 2 between the MDER values). However, even though the color points are essentially the same (using 10° color matching functions), it appears that human observers may be able to see color differences in some luminaires, such as e.g. panels and lens luminaires). Such observers seem to observe blueish or greenish light from the blue pump light sources and pinkish or purplish light from the blueish-cyan like light sources. Hence, previously the color points may be matched using the 10° CMF (color matching function; see also below), whereby the color points using 2° CMF appear to be very different. People might see that color difference when looking at the luminaires. This may be less desirable.

Hence, it is an aspect of the invention to provide an alternative light generating system, which preferably further at least partly obviates one or more of above-described drawbacks. The present invention may have as object to overcome or ameliorate at least one of the disadvantages of the prior art, or to provide a useful alternative.

The invention is defined by a light generating system in accordance with claim 1. Further preferred embodiments are defined in the dependent claims.

According to a first aspect, the invention provides a light generating system ("system") comprising a first light generating device and a second light generating device. Especially, the first light generating device may be configured to generate first device light having a primary first color point ($x_{2\_1}$,$y_{2\_1}$) calculated using CIE 1931 2° color matching functions, and a secondary first color point ($x_{10\_1}$,$y_{10\_1}$) calculated using CIE 1964 10° color matching functions. **In** specific embodiments, the first light generating device may comprise a first (solid state) light source, configured to generate first light source light, and a first luminescent material, configured to convert at least part of the first light source light into first luminescent material light. Further, especially, the first light source light may have a first dominant wavelength ($\lambda_{d1}$) selected from the wavelength range of 440-470 nm. Especially, the first device light may comprise at least part of the first light source light and at least part of the first luminescent material light. Especially, the second light generating device may be configured to generate second device light having a primary second color point ($x_{2\_2}$,$y_{2\_2}$) calculated using CIE 1931 2° color matching functions, and a secondary second color point ($x_{10\_2}$,$y_{10\_2}$) calculated using CIE 1964 10° color matching functions. **In** specific embodiments, the second light generating device may comprise a second (solid state) light source, configured to generate second light source light, and a second luminescent material, configured to convert at least part of the second light source light into second luminescent material light. Further, especially the second light source light may have a second dominant wavelength ($\lambda_{d2}$) selected from the wavelength range of 470-500 nm. Especially, the second device light may comprise at least part of the second light source light and at least part of the second luminescent material light. **In** specific embodiments, one of the first device light and the second device light may have a relatively higher contribution in the 470-500 nm wavelength range, relative to a total spectral power distribution in the visible wavelength range (of the respective device light), than the other one of the first device light and the second device light. In specific embodiments, a difference between the primary first color point ($x_{2\_1}$,$y_{2\_1}$) and the primary second color point ($x_{2\_2}$,$y_{2\_2}$) may be within 10 SDCM (standard deviation of color matching), more especially within about 6 SDCM. Further, in specific embodiments combined light, comprising a combination of the first device light and the second device light, may comprise: (i) a secondary color point ($x_{10\_sum}$,$y_{10\_sum}$), calculated using CIE 1964 10° color matching functions, may be configured within 10 SDCM, more especially within 6 SDCM from the black body locus (BBL), and (ii) a combined light correlated color temperature ($T_{cc}$) may be selected from the range of 2700-10000 K, more especially 3000-8000 K. In embodiments, the invention provides a light generating system comprising a first light generating device and a second light generating device, wherein: (A) the first light generating device is configured to generate first device light having a primary first color point $(x_{2\_1},y_{2\_1})$ calculated using CIE 1931 2° color matching functions, and a secondary first color point $(x_{10\_1},y_{10\_1})$ calculated using CIE 1964 10° color matching functions; (B) the second light generating device is configured to generate second device light having a primary second color point $(x_{2\_2},y_{2\_2})$ calculated using CIE 1931 2° color matching functions, and a secondary second color point $(x_{10\_2},y_{10\_2})$ calculated using CIE 1964 10° color matching functions; (C) one of the first device light and the second device light has a relatively higher contribution in the 470-500 nm wavelength range, relative to a total spectral power distribution in the visible wavelength range, than the other one of the first device light and the second device light; (D) a difference between the primary first color point $(x_{2\_1},y_{2\_1})$ and the primary second color point $(x_{2\_2},y_{2\_2})$ is within 6 SDCM; and (E) combined light, comprising a combination of the first device light and the second device light, comprises: (i) a secondary color point $(x_{10\_sum},y_{10\_sum})$, calculated using CIE 1964 10° color matching functions, configured within 6 SDCM from the black body locus, and (ii) a combined light correlated color temperature $(T_{cc})$ selected from the range of 3000-8000 K. Hence, in specific embodiments the invention provides a light generating system comprising a first light generating device and a second light generating device, wherein: (A) the first light generating device is configured to generate first device light having a primary first color point $(x_{2\_1},y_{2\_1})$ calculated using CIE 1931 2° color matching functions, and a secondary first color point $(x_{10\_1},y_{10\_1})$ calculated using CIE 1964 10° color matching functions; wherein the first light generating device comprises a first (solid state) light source, configured to generate first light source light, and a first luminescent material, configured to convert at least part of the first light source light into first luminescent material light, wherein the first light source light has a first dominant wavelength $(\lambda_{d1})$ selected from the wavelength range of 440-470 nm, and wherein the first device light comprises at least part of the first light source light and at least part of the first luminescent material light; (B) the second light generating device is configured to generate second device light having a primary second color point $(x_{2\_2},y_{2\_2})$ calculated using CIE 1931 2° color matching functions, and a secondary second color point $(x_{10\_2},y_{10\_2})$ calculated using CIE 1964 10° color matching functions; wherein the second light generating device comprises a second (solid state) light source, configured to generate second light source light, and a second luminescent material, configured to convert at least part of the second light source light into second luminescent material light, wherein the second light source light has a second dominant wavelength $(\lambda_{d2})$ selected from the wavelength range of 470-500 nm, and wherein the second device light comprises at least part of the second light source light and at least part of the second luminescent material light; (C) one of the first device light and the second device light has a relatively higher contribution in the 470-500 nm wavelength range, relative to a total spectral power distribution in the visible wavelength range, than the other one of the first device light and the second device light; (D) a difference between the primary first color point $(x_{2\_1},y_{2\_1})$ and the primary second color point $(x_{2\_2},y_{2\_2})$ is within 6 SDCM; and (E) combined light, comprising a combination of the first device light and the second device light, comprises: (i) a secondary color point $(x_{10\_sum},y_{10\_sum})$, calculated using CIE 1964 10° color matching functions, configured within 6 SDCM from the black body locus, and (ii) a combined light correlated color temperature $(T_{cc})$ selected from the range of 3000-8000 K.

With such light generating system, essentially no color difference can be observed when looking at the luminaire (2°), and "normal" white light may be observed in the room (10°) (especially without undesired color effects).

As indicated above, the invention provides a light generating system comprising a first light generating device and a second light generating device. Note that the term "first light generating device" may refer to one or more first light generating devices. Likewise, the term "second light generating device" may refer to one or more second light generating devices. Hence, the system may comprise one or more first light generating devices and one or more second light generating devices. Here below, some aspects in relation to light generating devices are described.

A light generating device may especially be configured to generate device light. Especially, the light generating device may comprise a light source. The light source may especially be configured to generate light source light. In embodiments, the device light may essentially consist of the light source light. In other embodiments, the device light may essentially consist of converted light source light. In yet other embodiments, the device light may comprise (unconverted) light source light and converted light source light. Light source light may be converted with a luminescent material into luminescent material light and/or with an upconverter into upconverted light (see also below). The term "light generating device" may also refer to a plurality of light generating devices which may provide device light having essentially the same spectral power distributions. In specific embodiments, the term "light generating device" may also refer to a plurality of light generating devices which may provide device light having different spectral power distributions.

The light generating device may comprise one or more light sources, more especially one or more solid state light sources. Further, the light generating device may comprise optics. Light, i.e. light source light (from the one or more light sources), escaping from the one or more light sources may in embodiments be beam shaped via the optics. Device light may especially comprise the light source light. More especially, the device light may essentially consist of the (light source) light of the one or more light sources.

The term "light source" may in principle relate to any light source known in the art. It may be a conventional (tungsten) light bulb, a low pressure mercury lamp, a high pressure mercury lamp, a fluorescent lamp, an LED (light emissive diode). In a specific embodiment, the light source comprises a solid state LED light source (such as an LED or laser diode (or "diode laser")). The term "light source" may also relate to a plurality of light sources, such as 2-2000 (solid state) LED light sources. Hence, the term LED may also refer to a plurality of LEDs. Further, the term "light source" may in embodiments also refer to a so-called chip-on-board (COB) light source. The term "COB" especially refers to LED chips in the form of a semiconductor chip that is neither encased nor connected but directly mounted onto a substrate, such as a PCB. Hence, a plurality of light emitting semiconductor light sources may be configured on the same substrate. In embodiments, a COB is a multi LED chip configured together as a single lighting module. The term "light source" may also refer to a chip scaled package (CSP). A CSP may comprise a single solid state die with provided thereon a luminescent material comprising layer. The term "light source" may also refer to a midpower package. A midpower package may comprise one or more solid state die(s). The die(s) may be covered by a luminescent material comprising layer. The die dimensions may be equal to or smaller than 2 mm, such as in the range of e.g. 0.2-2 mm. Hence, in embodiments the light source comprises a solid state light source. Further, in specific embodiments, the light source comprises a chip scale packaged LED. Herein, the term "light source" may also especially refer to a small solid state light source, such as having a mini size or micro size. For instance, the light sources may comprise one or more of mini LEDs and micro LEDs. Especially, in embodiment the light sources comprise micro LEDs or "microLEDs" or "$\mu$LEDs''. Herein, the term mini size or mini LED especially indicates to solid state light sources having dimensions, such as die dimension, especially length and width, selected from the range of 100 $\mu$m - 1 mm. Herein, the term $\mu$ size or micro LED especially indicates to solid state light sources having dimensions, such as die dimension, especially length and width, selected from the range of 100 $\mu$m and smaller.

The light source may have a light escape surface. Referring to conventional light sources such as light bulbs or fluorescent lamps, it may be an outer surface of a glass or a quartz envelope. For LED's it may for instance be the LED die, or when a resin is applied to the LED die, the outer surface of the resin. In principle, it may also be the terminal end of a fiber. The term escape surface especially relates to that part of the light source, where the light actually leaves or escapes from the light source. The light source is configured to provide a beam of light. This beam of light (thus) escapes from the light exit surface of the light source.

Likewise, a light generating device may comprise a light escape surface, such as an end window. Further, likewise a light generating system may comprise a light escape surface, such as an end window.

The term "light source" may refer to a semiconductor light-emitting device, such as a light emitting diode (LEDs), a resonant cavity light emitting diode (RCLED), a vertical cavity laser diode (VCSELs), an edge emitting laser, etc. The term "light source" may also refer to an organic light-emitting diode (OLED), such as a passive-matrix (PMOLED) or an active-matrix (AMOLED). In a specific embodiment, the light source comprises a solid-state light source (such as an LED or laser diode). In an embodiment, the light source comprises an LED (light emitting diode). The terms "light source" or "solid state light source" may also refer to a superluminescent diode (SLED). The term LED may also refer to a plurality of LEDs. The term "light source" may also relate to a plurality of (essentially identical (or different)) light sources, such as 2-2000 solid state light sources. In embodiments, the light source may comprise one or more micro-optical elements (array of micro lenses) downstream of a single solid-state light source, such as an LED, or downstream of a plurality of solid-state light sources (i.e. e.g. shared by multiple LEDs). In embodiments, the light source may comprise an LED with on-chip optics. In embodiments, the light source comprises pixelated single LEDs (with or without optics) (offering in embodiments on-chip beam steering).

The terms "upstream" and "downstream" relate to an arrangement of items or features relative to the propagation of the light from a light generating means (here the especially the light source), wherein relative to a first position within a beam of light from the light generating means, a second position in the beam of light closer to the light generating means is "upstream", and a third position within the beam of light further away from the light generating means is "downstream".

In embodiments, the light source may be configured to provide primary radiation, which is used as such, such as e.g. a blue light source, like a blue LED, or a green light source, such as a green LED, and a red light source, such as a red LED. Such LEDs, which may not comprise a luminescent material ("phosphor") may be indicated as direct color LEDs. In other embodiments, however, the light source may be configured to provide primary radiation and part of the primary radiation is converted into secondary radiation. Secondary radiation may be based on conversion by a luminescent material. The secondary radiation may therefore also be indicated as luminescent material radiation. The luminescent material may in embodiments be comprised by the light source, such as an LED with a luminescent material layer or dome comprising luminescent material. Such LEDs may be indicated as phosphor converted LEDs or PC LEDs (phosphor converted LEDs). In other embodiments, the luminescent material may be configured at some distance ("remote") from the light source, such as an LED with a luminescent material layer not in physical contact with a die of the LED. Hence, in specific embodiments the light source may be a light source that during operation emits at least light at wavelength selected from the range of 380-470 nm. However, other wavelengths may also be possible. This light may partially be converted by the luminescent material.

In embodiments, the light generating device may comprise a luminescent material. In embodiments, the light generating device may comprise a PC LED. In other embodiments, the light generating device may comprise a direct LED (i.e. no phosphor). In embodiments, the light generating device may comprise a laser device, like a laser diode. In embodiments, the light generating device may comprise a superluminescent diode. Hence, in specific embodiments, the light source may be selected from the group of laser diodes and superluminescent diodes. In other embodiments, the light source may comprise an LED.

The light source may especially be configured to generate light source light having an optical axis (O), (a beam shape,) and a spectral power distribution. The light source light may in embodiments comprise one or more bands, having band widths as known for lasers.

The term "light source" may (thus) refer to a light generating element as such, like e.g. a solid state light source, or e.g. to a package of the light generating element, such as a solid state light source, and one or more of a luminescent material comprising element and (other) optics, like a lens, a collimator. A light converter element ("converter element" or "converter") may comprise a luminescent material comprising element. For instance, a solid state light source as such, like a blue LED, is a light source. A combination of a solid state light source (as light generating element) and a light converter element, such as a blue LED and a light converter element, optically coupled to the solid state light source, may also be a light source (but may also be indicated as light generating device). Hence, a white LED is a light source (but may e.g. also be indicated as (white) light generating device).

The term "light source" herein may also refer to a light source comprising a solid state light source, such as an LED or a laser diode or a superluminescent diode.

The term "light source" may (thus) in embodiments also refer to a light source that is (also) based on conversion of light, such as a light source in combination with a luminescent converter material. Hence, the term "light source" may also refer to a combination of an LED with a luminescent material configured to convert at least part of the LED radiation, or to a combination of a (diode) laser with a luminescent material configured to convert at least part of the (diode) laser radiation. In embodiments, the term "light source" may also refer to a combination of a light source, like an LED, and an optical filter, which may change the spectral power distribution of the light generated by the light source. Especially, the term "light generating device" may be used to address a light source and further (optical components), like an optical filter and/or a beam shaping element, etc.

The phrases "different light sources" or "a plurality of different light sources", and similar phrases, may in embodiments refer to a plurality of solid-state light sources selected from at least two different bins. Likewise, the phrases "identical light sources" or "a plurality of same light sources", and similar phrases, may in embodiments refer to a plurality of solid-state light sources selected from the same bin.

The term "solid state light source", or "solid state material light source", and similar terms, may especially refer to semiconductor light sources, such as a light emitting diode (LED), a laser diode, or a superluminescent diode.

Herein, especially both the first light generating device and the second light generating device comprise a solid state light source. More especially, in embodiments both the first light generating device and the second light generating device may comprise a (solid state) light source, configured to generate light source light, and a luminescent material, configured to convert at least part of the light source light into luminescent material light. The spectral power distributions of the first device light and the second device light may however differ. Embodiments of the two light generating devices are described in more detail below.

Especially, the first light generating device may be configured to generate first device light having a primary first color point ($x_{2\_1},y_{2\_1}$) calculated using 2° color matching functions, and a secondary first color point ($x_{10\_1},y_{10\_1}$) calculated using CIE 1964 10° color matching functions. As also indicated above, it appears that the primary first color point ($x_{2\_1},y_{2\_1}$) and the secondary first color point ($x_{10\_1},y_{10\_1}$) may substantially differ, like several SDCMs.

Hence, especially, the color points (e.g. the primary first color point , the primary second color point, the secondary first color point, the secondary second color point, u', v') are based on the 2° or 10° color matching functions according to ISO/CIE 11664-1:2019-06 Colorimetry - Part 1: CIE standard colorimetric observers, CIE 1931 (table 1, 2° CMF), CIE 1964 (table 2, 10°), and ISO/CIE 11664-5:2016-09-01 Colorimetry - Part 5: CIE 1976 L*u*v* color space and u', v' uniform chromaticity scale diagram (see e.g. 4.1). The 2° color matching function may be defined as a numerical description of the chromatic response of the CIE 1931 2° standard observer. The 10° color matching function may be defined as a numerical description of the chromatic response of the CIE 1964 10° standard observer. The CIE 1931 2° and CIE 1963 10° color matching functions are herein also indicated as "2° CMFs" and "10° CMFs", respectively, and similar phrases, like simply "2°" and "10°", respectively.

In specific embodiments, the first light generating device may comprise a first (solid state) light source, configured to generate first light source light, and a first luminescent material, configured to convert at least part of the first light source light into first luminescent material light. Especially, the first light source may comprise a light emitting diode, though other (solid state) light source options may also be possible. Further, the term "first luminescent material" may refer to one or more (different) luminescent materials. Further, especially, the first light source light may have a first dominant wavelength ($\lambda_{d1}$) selected from the wavelength range of 440-470 nm. Hence, in embodiments the first light source may especially have intensity in the blue wavelength range and may provide blue light. The term "dominant wavelength", known in the art, may refer to the wavelength of the monochromatic stimulus that, when additively mixed in suitable proportions with the specified achromatic stimulus, matches the color stimulus considered.

Further, especially, the first device light may comprise at least part of the first light source light and at least part of the first luminescent material light. More especially, the first device light may be white light or whitish light, having a correlated color temperature selected (CCT1) from the range of 2700-10000 K, more especially 3000-8000 K, such as over 3000 K, like at least about 3200 K.

Especially, the second light generating device may be configured to generate second device light having a primary second color point $(x_{2\_2}, y_{2\_2})$ calculated using CIE 1931 2° color matching functions, and a secondary second color point $(x_{10\_2}, y_{10\_2})$ calculated using CIE 1964 10° color matching functions. As also indicated above, it appears that the primary second color point $(x_{2\_2}, y_{2\_2})$ and the secondary second color point $(x_{10\_2}, y_{10\_2})$ may substantially differ, like several SDCMs. Especially, this may apply to luminescent material comprising light generating devices wherein the luminescent material is pumped with (solid state) light source light in the 470-500 nm wavelength range. The difference in primary second color point $(x_{2\_2}, y_{2\_2})$ and the secondary second color point $(x_{10\_2}, y_{10\_2})$ may be larger than the difference in the primary first color point $(x_{2\_1}, y_{2\_1})$ and the secondary first color point $(x_{10\_1}, y_{10\_1})$.

In specific embodiments, the second light generating device may comprise a second (solid state) light source, configured to generate second light source light, and a second luminescent material, configured to convert at least part of the second light source light into second luminescent material light. Especially, the second light source may comprise a light emitting diode, though other (solid state) light source options may also be possible. Further, the term "second luminescent material" may refer to one or more (different) luminescent materials.

Especially, the first luminescent material and the second luminescent material may be different luminescent materials, even though in specific embodiments they may at least partly be based on luminescent materials of the same class(es). Hence, the spectral power distribution of the first luminescent material light and the second luminescent material light may differ. Hence, the spectral power distributions of the first luminescent material light and the second luminescent material light will not be identical.

In specific embodiments, colors or color points of a first type of light and a second type of light may be different when the respective color points of the first type of light and the second type of light differ with at least 0.01 for u' and/or with at least 0.01 for v', even more especially at least 0.02 for u' and/or with at least 0.02 for v'. In yet more specific embodiments, the respective color points of first type of light and the second type of light may differ with at least 0.03 for u' and/or with at least 0.03 for v'. Here, u' and v' are color coordinates of the light in the CIE 1976 UCS (uniform chromaticity scale) diagram. Spectral power distributions of different sources of light having centroid wavelengths differing least 10 nm, such as at least 20 nm, or even at least 30 nm may be considered different spectral power distributions, e.g. different colors. In general, the differences in centroid wavelengths will not be larger than about 400 nm, such as not more than 350 nm. The color points indicated with u',v' especially refer to the CIE 1976 color points (see ISO CIE 11664-5: 2016-09-01 Colorimetry - Part5: CIE 1976 L*u*v* color space and u', v' uniform chromaticity scale diagram).

The term "centroid wavelength", also indicated as $\lambda c$, is known in the art, and refers to the wavelength value where half of the light energy is at shorter and half the energy is at longer wavelengths; the value is stated in nanometers (nm). It is the wavelength that divides the integral of a spectral power distribution into two equal parts as expressed by the formula $\lambda c = \Sigma \lambda * I(\lambda) / (\Sigma I(\lambda))$, where the summation is over the wavelength range of interest, and $I(\lambda)$ is the spectral energy density (i.e. the integration of the product of the wavelength and the intensity over the emission band normalized to the integrated intensity). The centroid wavelength may e.g. be determined at operation conditions.

Further, especially the second light source light may have a second dominant wavelength $(\lambda_{d2})$ selected from the wavelength range of 470-500 nm. Hence, in embodiments the second light source may especially have intensity in the blue-cyan like wavelength range and may provide blueish-cyan like light. Herein, the terms "blueish-cyan like light" and "blue-cyan like" may especially refer to light having one or more wavelengths in the 470-520 nm, and especially light having spectral power at one or more wavelengths in the 470-500 nm wavelength range.

Hence, the spectral power distributions of the first light source light and the second light source light will not be identical and may e.g. only partly overlap (or even not overlap). In specific embodiments, the dominant wavelengths of the first light source light and the second light source light may substantially differ, like with at least 5 nm, more especially at least 10 nm. Hence, in embodiments $|(\lambda_{d1}-\lambda_{d2})| \geq 10$ nm, such as more than 10 nm. Especially, in embodiments $10$ nm $\leq |(\lambda_{d1}-\lambda_{d2})| \leq 60$ nm, more especially $15$ nm $\leq |(\lambda_{d1}-\lambda_{d2})| \leq 50$ nm, such as $20$ nm $\leq |(\lambda_{d1}-\lambda_{d2})| \leq 40$ nm. As may be clear from the above, especially $\lambda_{d1} < \lambda d_2$.

Especially, the second device light may comprise at least part of the second light source light and at least part of the second luminescent material light. More especially, the second device light may be white light or whitish light, having a correlated color temperature (CCT2) selected from the range of 2700-10000 K, more especially 3000-8000 K, such as over 3000 K, like at least about 3200 K.

As will be elucidated below, especially the first device light and the second device light may have substantially identical correlated color temperatures. However, the spectral power distributions of the first device light and the second device light may not fully overlap. In embodiments, $|(CCT1-CCT2)| \leq 1000$ K, such as $|(CCT1-CCT2)| \leq 500$ K.

In specific embodiments, one of the first device light and the second device light may have a relatively higher contribution in the 470-500 nm wavelength range, relative to a total spectral power distribution in the visible wavelength range (of the respective device light), than the other one of the first device light and the second device light. As indicated above, especially herein the second device light is herein also indicated as "blue-cyan like enriched" or "blue-cyan like enriched device light".

Especially, herein the second device light may have a relatively higher contribution in the 470-500 nm wavelength range, relative to a total spectral power distribution in the visible wavelength range (of the second device light), than the first device light. This may in embodiments especially be due to the cyan shifted emission of the second light source.

As indicated above, with the herein defined choices, non-blue-cyan like enriched and blue-cyan like enriched pump light sources may be used to provide white light which may also be perceived as white light, with less undesired color effects than would a state of the art solution be chosen. Basically, a plurality of measures are taken: the 2° color points should not be too far away from each other, and the 10° color point of the sum of the light of the first device light and the second device light should not be too far away from the BBL, even though the individual 10° color points may be (but are not necessarily) relatively far away from the BBL. Further, in embodiments the color points of the first device light and the second device light, more especially the color point of the combined first device light and second device light, should desirably be chosen in a specific CCT range.

Hence, in specific embodiments, a difference between the primary first color point $(x_{2\_1}, y_{2\_1})$ and the primary second color point $(x_{2\_2}, y_{2\_2})$ may be within 10 SDCM, more especially within about 6 SDCM. Further, in specific embodiments combined light, comprising a combination of the first device light and the second device light, may comprise: (i) a secondary color point $(x_{10\_sum}, y_{10\_sum})$, calculated using CIE 1964 10° color matching functions, may be configured within 10 SDCM, more especially within 6 SDCM from the black body locus, and (ii) a combined light correlated color temperature $(T_{cc})$ may be selected from the range of 2700-10000 K, more especially 3000-8000 K. In specific embodiments, the combined 10° color point may be on, below or above the BBL, especially in embodiments below the BBL. The term "combined light correlated color temperature" especially refer to the correlated color temperature of the combined light (i.e. of the combination of the first device light and the second device light).

Here below, some further embodiments will be described.

As indicated above, the first device light may be based on a blue pump light source and the second device light may be based on a blue-cyan like enriched pump light source. The device light generated with the latter light source may be enriched in the 470-500 nm area, relative to the device light generated with the former light source.

Especially, the first light generating device (110) may be configured to generate the first device light in the 440-500 nm wavelength range. At least 80% of the spectral power of the first device light may be within the 440-470 nm wavelength range within the 440-500 nm wavelength range. The second light generating device may be configured to generate the second device light in the 440-500 nm wavelength range. At least 80% of the spectral power of the second device light may be within the 470-500 nm wavelength range within the 440-500 nm wavelength range. Such that one of the first device light and the second device light has a relatively higher contribution in the 470-500 nm wavelength range, relative to a total spectral power distribution in the visible wavelength range, than the other one of the first device light and the second device light.

The first light generating device may be configured to generate the first device light having at least 60%, more especially at least 70%, yet even more especially at least 80% of its spectral power in the 440-500 nm wavelength range within the 440-470 nm wavelength range. For instance, in embodiments at least 80% of the spectral power of the first device light in the 440-500 nm wavelength range may be within the 440-470 nm wavelength range.

As can be derived hereof, in embodiments in the order of about at least 70%, more especially at least about 80%, such as at least about 85% of the spectral power of the first (solid state) light source light in the 380-780 nm wavelength range may be in the 440-470 nm wavelength range.

Further, in embodiments - the second light generating device may be configured to generate the second device light having at least 60%, more especially at least 70%, yet even more especially at least 80% of its spectral power in the 440-500 nm wavelength range within the 470-500 nm wavelength range. For instance, in embodiments at least 80% of the spectral power of the second device light in the 440-500 nm wavelength range may be within the 470-500 nm wavelength range.

As can be derived hereof, in embodiments in the order of about at least 70%, more especially at least about 80%, such as at least about 85% of the spectral power of the second (solid state) light source light in the 380-780 nm wavelength range may be in the 470-500 nm wavelength range. As indicated above, in embodiments $|(\lambda_{d1}-\lambda_{d2})| \geq 10$ nm, such as more than 10 nm.

The secondary first color point $(x_{10\_1}, y_{10\_1})$ calculated using CIE 1964 10° color matching functions may be above the black body locus, wherein the secondary second color point $(x_{10\_2}, y_{10\_2})$ calculated using CIE 1964 10° color matching functions may be below the black body locus, and wherein the secondary color point $(x_{10\_sum}, y_{10\_sum})$ calculated using CIE 1964 10° color matching functions may be configured within 6 SDCM from the black body locus.

As indicated above, the 10° color point of the sum of the light of the first device light and the second device light should not be too far away from the BBL, even though the individual 10° color points may be (but are not necessarily) relatively far away from the BBL. In embodiments, a difference between the secondary first color point $(x_{10\_1}, y_{10\_1})$ and the secondary second color point $(x_{10\_2}, y_{10\_2})$ may be at least 3 SDCM, such as at least 5 SDCM. In specific embodiments, a difference between the secondary first color point $(x_{10\_1}, y_{10\_1})$ and the secondary second color point $(x_{10\_2}, y_{10\_2})$ may be selected from the range of 3-50 SDCM, such as 5-40 SDCM. Further, especially in embodiments the secondary first color point $(x_{10\_1}, y_{10\_1})$ and the secondary second color point $(x_{10\_2}, y_{10\_2})$ are each configured within 25 SDCM, such as within 20 SDCM from the black body locus.

As indicated above, the combined light correlated color temperature $(T_{cc})$ may be selected from the range of 3000-8000 K. In specific embodiments, the combined light correlated color temperature $(T_{cc})$ may be selected from the range of 3000-6500 K. In specific embodiments, the combined light correlated color temperature $(T_{cc})$ may be selected from the range of 3000-5000 K.

As indicated above, the first light generating device and the second light generating device may each comprise one or more luminescent materials. Here below, first some general aspects in relation to luminescent materials are described.

The term "luminescent material" especially refers to a material that can convert first radiation, especially one or more of UV radiation and blue radiation, into second radiation. In general, the first radiation and second radiation have different spectral power distributions. Hence, instead of the term "luminescent material", also the terms "luminescent converter" or "converter" may be applied. In general, the second radiation has a spectral power distribution at larger wavelengths than the first radiation, which is the case in the so-called down-conversion. In specific embodiments, however the second radiation has a spectral power distribution with intensity at smaller wavelengths than the first radiation, which is the case in the so-called up-conversion.

In embodiments, the "luminescent material" may especially refer to a material that can convert radiation into e.g. visible and/or infrared light. For instance, in embodiments the luminescent material may be able to convert one or more of UV radiation and blue radiation, into visible light. The luminescent material may in specific embodiments also convert radiation into infrared radiation (IR). Hence, upon excitation with radiation, the luminescent material emits radiation. In general, the luminescent material will be a down converter, i.e. radiation of a smaller wavelength is converted into radiation with a larger wavelength ($\lambda_{ex} < \lambda_{em}$), though in specific embodiments the luminescent material may comprise up-converter luminescent material, i.e. radiation of a larger wavelength is converted into radiation with a smaller wavelength ($\lambda_{ex} > \lambda_{em}$).

In embodiments, the term "luminescence" may refer to phosphorescence. In embodiments, the term "luminescence" may also refer to fluorescence. Instead of the term "luminescence", also the term "emission" may be applied. Hence, the terms "first radiation" and "second radiation" may refer to excitation radiation and emission (radiation), respectively. Likewise, the term "luminescent material" may in embodiments refer to phosphorescence and/or fluorescence.

The term "luminescent material" may also refer to a plurality of different luminescent materials. Examples of possible luminescent materials are indicated below. Hence, the term "luminescent material" may in specific embodiments also refer to a luminescent material composition. Instead of the term "luminescent material" also the term "phosphor" may be applied. These terms are known to the person skilled in the art.

In embodiments, luminescent materials are selected from garnets and nitrides, especially doped with trivalent cerium or divalent europium, respectively. The term "nitride" may also refer to oxynitride or nitridosilicate, etc. Alternatively or additionally, the luminescent material(s) may be selected from silicates, especially doped with divalent europium.

In specific embodiments the luminescent material comprises a luminescent material of the type $A_3B_5O_{12}$:Ce, wherein A in embodiments comprises one or more of Y, La, Gd, Tb and Lu, especially (at least) one or more of Y, Gd, Tb and Lu, and wherein B in embodiments comprises one or more of Al, Ga, In and Sc. Especially, A may comprise one or more of Y, Gd and Lu, such as especially one or more of Y and Lu. Especially, B may comprise one or more of Al and Ga, more especially at least Al, such as essentially entirely Al. Hence, especially suitable luminescent materials are cerium comprising garnet materials. Embodiments of garnets especially include $A_3B_5O_{12}$ garnets, wherein A comprises at least yttrium or lutetium and wherein B comprises at least aluminum. Such garnets may be doped with cerium (Ce), with praseodymium (Pr) or a combination of cerium and praseodymium; especially however with Ce. Especially, B may comprise aluminum (Al); however, in addition to aluminum, B may also partly comprise gallium (Ga) and/or scandium (Sc) and/or indium (In), especially up to about 20% of B, more especially up to about 10 % of B (i.e. the B ions essentially consist of 90 or more mole % of Al and 10 or less mole % of one or more of Ga, Sc and In); B may especially comprise up to about 10% gallium. In another variant, B and O may at least partly be replaced by Si and N. The element A may especially be selected from the group consisting of yttrium (Y), gadolinium (Gd), terbium (Tb) and lutetium (Lu). Further, Gd and/or Tb are especially only present up to an amount of about 20% of A. In a specific embodiment, the garnet luminescent material comprises $(Y_{1-x}Lu_x)_3B_5O_{12}$:Ce, wherein x is equal to or larger than 0 and equal to or smaller than 1. The term ":Ce", indicates that part of the metal ions (i.e. in the garnets: part of the "A" ions) in the luminescent material is replaced by Ce. For instance, in the case of $(Y_{1-x}Lu_x)_3Al_5O_{12}$:Ce, part of Y and/or Lu is replaced by Ce. This is known to the person skilled in the art. Ce will replace A in general for not more than 10%; in general, the Ce concentration will be in the range of 0.1 to 4%, especially 0.1 to 2% (relative to A). Assuming 1% Ce and 10% Y, the full correct formula could be $(Y_{0.1}Lu_{0.89}Ce_{0.01})_3Al_5O_{12}$. Ce in garnets is substantially or only in the trivalent state, as is known to the person skilled in the art.

In embodiments, the luminescent material (thus) comprises $A_3B_5O_{12}$ wherein in specific embodiments at maximum 10% of B-O may be replaced by Si-N.

In specific embodiments the luminescent material comprises $(Y_{x1}A'_{x2}Ce_{x3})_3(Al_{y1}B'_{y2})_5O_{12}$, wherein $x1+x2+x3=1$, wherein $x3>0$, wherein $0<x2+x3\leq0.2$, wherein $y1+y2=1$, wherein especially $0\leq y2\leq0.2$, wherein A' comprises one or more elements selected from the group consisting of lanthanides, and wherein B' comprises one or more elements selected from the group consisting of Ga, In and Sc. In embodiments, x3 is selected from the range of 0.001-0.1. In the present invention, especially $x1>0$, such as $>0.2$, like at least 0.8. Garnets with Y may provide suitable spectral power distributions.

In specific embodiments at maximum 10% of B-O may be replaced by Si-N. Here, B in B-O refers to one or more of Al, Ga, In and Sc (and O refers to oxygen); in specific embodiments B-O may refer to Al-O. As indicated above, in specific embodiments x3 may be selected from the range of 0.001-0.04. Especially, such luminescent materials may have a suitable spectral distribution (see however below), have a relatively high efficiency, have a relatively high thermal stability, and allow a high CRI (optionally in combination with (the) light of other sources of light as described herein). Hence, in specific embodiments A may be selected from the group consisting of Lu and Gd. Alternatively or additionally, B may comprise Ga. Hence, in embodiments the luminescent material comprises $(Y_{x1}(Lu,Gd)_{x2}Ce_{x3})_3(Al_{y1}Ga_{y2})_5O_{12}$, wherein Lu and/or Gd may be available. Even more especially, x3 is selected from the range of 0.001-0.1, wherein $0<x2+x3\leq0.1$, and wherein $0\leq y2\leq0.1$. Further, in specific embodiments, at maximum 1% of B-O may be replaced by SiN. Here, the percentage refers to moles (as known in the art); see e.g. also EP3149108. In yet further specific embodiments, the luminescent material comprises $(Y_{x1}Ce_{x3})_3Al_5O_{12}$, wherein $x1+x3=1$, and wherein $0<x3\leq0.2$, such as 0.001-0.1.

In specific embodiments, the light generating device may only include luminescent materials selected from the type of cerium comprising garnets. In even further specific embodiments, the light generating device includes a single type of luminescent materials, such as $(Y_{x1}A'_{x2}Ce_{x3})_3(Al_{y1}B'_{y2})_5O_{12}$. Hence, in specific embodiments the light generating device comprises luminescent material, wherein at least 85 weight%, even more especially at least about 90 wt.%, such as yet even more especially at least about 95 weight % of the luminescent material comprises $(Y_{x1}A'_{x2}Ce_{x3})_3(Al_{y1}B'_{y2})_5O_{12}$. Here, wherein A' comprises one or more elements selected from the group consisting of lanthanides, and wherein B' comprises one or more elements selected from the group consisting of Ga, In and Sc, wherein $x1+x2+x3=1$, wherein $x3>0$, wherein $0<x2+x3\leq0.2$, wherein $y1+y2=1$, wherein $0\leq y2\leq0.2$. Especially, x3 is selected from the range of 0.001-0.1. Note that in embodiments $x2=0$. Alternatively or additionally, in embodiments $y2=0$. In other embodiments, the light generating device may include one or more luminescent materials, wherein at least one is selected from the type of cerium comprising garnets.

In specific embodiments, A may especially comprise at least Y, and B may especially comprise at least Al.

Alternatively or additionally, the luminescent material may comprise a luminescent material of the type $A_3Si_6N_{11}:Ce^{3+}$, wherein A comprises one or more of Y, La, Gd, Tb and Lu, such as in embodiments one or more of La and Y.

In embodiments, the luminescent material may alternatively or additionally comprise one or more of $MS:Eu^{2+}$ and/or $M_2Si_5N_8:Eu^{2+}$ and/or $MAlSiN_3:Eu^{2+}$ and/or $Ca_2AlSi_3O_2N_5:Eu^{2+}$, etc., wherein M comprises one or more of Ba, Sr, and Ca, especially in embodiments at least Sr. Hence, in embodiments, the luminescent may comprise one or more materials selected from the group consisting of (Ba,Sr,Ca)S:Eu, (Ba,Sr,Ca)AlSiN$_3$:Eu and (Ba,Sr,Ca)$_2$Si$_5$N$_8$:Eu. In these compounds, europium (Eu) is substantially or only divalent, and replaces one or more of the indicated divalent cations. In general, Eu will not be present in amounts larger than 10% of the cation; its presence will especially be in the range of about 0.5 to 10%, more especially in the range of about 0.5 to 5% relative to the cation(s) it replaces. The term ":Eu", indicates that part of the metal ions is replaced by Eu (in these examples by $Eu^{2+}$). For instance, assuming 2% Eu in CaAlSiN$_3$:Eu, the correct formula could be $(Ca_{0.98}Eu_{0.02})AlSiN_3$. Divalent europium will in general replace divalent cations, such as the above divalent alkaline earth cations, especially Ca, Sr, or Ba. The material (Ba,Sr,Ca)S:Eu can also be indicated as MS:Eu, wherein M is one or more elements selected from the group consisting of barium (Ba), strontium (Sr) and calcium (Ca); especially, M comprises in this compound calcium or strontium, or calcium and strontium, more especially calcium. Here, Eu is introduced and replaces at least part of M (i.e. one or more of Ba, Sr, and Ca). Further, the material (Ba,Sr,Ca)$_2$Si$_5$N$_8$:Eu can also be indicated as M$_2$Si$_5$N$_8$:Eu, wherein M is one or more elements selected from the group consisting of barium (Ba), strontium (Sr) and calcium (Ca); especially, M comprises in this compound Sr and/or Ba. In a further specific embodiment, M consists of Sr and/or Ba (not taking into account the presence of Eu), especially 50 to 100%, more especially 50 to 90% Ba and 50 to 0%, especially 50 to 10% Sr, such as Ba$_{1.5}$Sr$_{0.5}$Si$_5$N$_8$:Eu (i.e. 75 % Ba; 25% Sr). Here, Eu is introduced and replaces at least part of M, i.e. one or more of Ba, Sr, and Ca). Likewise, the material (Ba,Sr,Ca)AlSiN$_3$:Eu can also be indicated as MAlSiN$_3$:Eu, wherein M is one or more elements selected from the group consisting of barium (Ba), strontium (Sr) and calcium (Ca); especially, M comprises in this compound calcium or strontium, or calcium and strontium, more especially calcium. Here, Eu is introduced and replaces at least part of M (i.e. one or more of Ba, Sr, and Ca). Eu in the above indicated luminescent materials is substantially or only in the divalent state, as is known to the person skilled in the art.

In embodiments, a red luminescent material may comprise one or more materials selected from the group consisting of (Ba,Sr,Ca)S:Eu, (Ba,Sr,Ca)AlSiN$_3$:Eu and (Ba,Sr,Ca)$_2$Si$_5$N$_8$:Eu. In these compounds, europium (Eu) is substantially or only divalent, and replaces one or more of the indicated divalent cations. In general, Eu will not be present in amounts larger than 10% of the cation; its presence will especially be in the range of about 0.5 to 10%, more especially in the range of about 0.5 to 5% relative to the cation(s) it replaces. The term ":Eu", indicates that part of the metal ions is replaced by Eu (in these examples by Eu$^{2+}$). For instance, assuming 2% Eu in CaAlSiN$_3$:Eu, the correct formula could be (Ca$_{0.98}$Eu$_{0.02}$)AlSiN$_3$. Divalent europium will in general replace divalent cations, such as the above divalent alkaline earth cations, especially Ca, Sr or Ba.

The material (Ba,Sr,Ca)S:Eu can also be indicated as MS:Eu, wherein M is one or more elements selected from the group consisting of barium (Ba), strontium (Sr) and calcium (Ca); especially, M comprises in this compound calcium or strontium, or calcium and strontium, more especially calcium. Here, Eu is introduced and replaces at least part of M (i.e. one or more of Ba, Sr, and Ca).

Further, the material (Ba,Sr,Ca)$_2$Si$_5$N$_8$:Eu can also be indicated as M$_2$Si$_5$N$_8$:Eu, wherein M is one or more elements selected from the group consisting of barium (Ba), strontium (Sr) and calcium (Ca); especially, M comprises in this compound Sr and/or Ba. In a further specific embodiment, M consists of Sr and/or Ba (not taking into account the presence of Eu), especially 50 to 100%, more especially 50 to 90% Ba and 50 to 0%, especially 50 to 10% Sr, such as Ba$_{1.5}$Sr$_{0.5}$Si$_5$N$_8$:Eu (i.e. 75 % Ba; 25% Sr). Here, Eu is introduced and replaces at least part of M, i.e. one or more of Ba, Sr, and Ca).

Likewise, the material (Ba,Sr,Ca)AlSiN$_3$:Eu can also be indicated as MAlSiN$_3$:Eu, wherein M is one or more elements selected from the group consisting of barium (Ba), strontium (Sr) and calcium (Ca); especially, M comprises in this compound calcium or strontium, or calcium and strontium, more especially calcium. Here, Eu is introduced and replaces at least part of M (i.e. one or more of Ba, Sr, and Ca).

Eu in the above indicated luminescent materials is substantially or only in the divalent state, as is known to the person skilled in the art.

Blue luminescent materials may comprise YSO (Y$_2$SiO$_5$:Ce$^{3+}$), or similar compounds, or BAM (BaMgAl$_{10}$O$_{17}$:Eu$^{2+}$), or similar compounds.

The term "luminescent material" herein especially relates to inorganic luminescent materials.

Alternatively or additionally, also other luminescent materials may be applied. For instance quantum dots and/or organic dyes may be applied and may optionally be embedded in transmissive matrices like e.g. polymers, like PMMA, or polysiloxanes, etc. etc.

Quantum dots are small crystals of semiconducting material generally having a width or diameter of only a few nanometers. When excited by incident light, a quantum dot emits light of a color determined by the size and material of the crystal. Light of a particular color can therefore be produced by adapting the size of the dots. Most known quantum dots with emission in the visible range are based on cadmium selenide (CdSe) with a shell such as cadmium sulfide (CdS) and zinc sulfide (ZnS). Cadmium free quantum dots such as indium phosphide (InP), and copper indium sulfide (CuInS$_2$) and/or silver indium sulfide (AgInS$_2$) can also be used. Quantum dots show very narrow emission band and thus they show saturated colors. Furthermore the emission color can easily be tuned by adapting the size of the quantum dots. Any type of quantum dot known in the art may be used in the present invention. However, it may be preferred for reasons of environmental safety and concern to use cadmium-free quantum dots or at least quantum dots having a very low cadmium content.

Instead of quantum dots or in addition to quantum dots, also other quantum confinement structures may be used. The term "quantum confinement structures" should, in the context of the present application, be understood as e.g. quantum wells, quantum dots, quantum rods, tripods, tetrapods, or nanowires, etcetera.

Organic phosphors can be used as well. Examples of suitable organic phosphor materials are organic luminescent materials based on perylene derivatives, for example compounds sold under the name Lumogen® by BASF. Examples of suitable compounds include, but are not limited to, Lumogen® Red F305, Lumogen® Orange F240, Lumogen® Yellow F083, and Lumogen® F170.

Different luminescent materials may have different spectral power distributions of the respective luminescent material light. Alternatively or additionally, such different luminescent materials may especially have different color points (or dominant wavelengths).

As indicated above, other luminescent materials may also be possible. Hence, in specific embodiments the luminescent material is selected from the group of divalent europium containing nitrides, divalent europium containing oxynitrides, divalent europium containing silicates, cerium comprising garnets, and quantum structures. Quantum structures may e.g. comprise quantum dots or quantum rods (or other quantum type particles) (see above). Quantum structures may also comprise quantum wells. Quantum structures may also comprise photonic crystals.

Especially, the first luminescent material may comprise a broad band emitter, have spectral power in the green-yellow wavelength range. The phrase "in the green-yellow wavelength range", and similar phrases, may indicate spectral power at one or more wavelengths in the green wavelength range and/or spectral power at one or more wavelengths in the yellow wavelength range (see also below).

The terms "violet light" or "violet emission", and similar terms, may especially relate to light having a wavelength in the range of about 380-440 nm. In specific embodiments, the violet light may have a centroid wavelength in the 380-440 nm range. The terms "blue light" or "blue emission", and similar terms, may especially relate to light having a wavelength in the range of about 440-490 nm (including some violet and cyan hues). In specific embodiments, the blue light may have a centroid wavelength in the 440-490 nm range. The terms "green light" or "green emission", and similar terms, may especially relate to light having a wavelength in the range of about 490-560 nm. In specific embodiments, the green light may have a centroid wavelength in the 490-560 nm range. The terms "yellow light" or "yellow emission", and similar terms, may especially relate to light having a wavelength in the range of about 560-590 nm. In specific embodiments, the yellow light may have a centroid wavelength in the 560-590 nm range. The terms "orange light" or "orange emission", and similar terms, may especially relate to light having a wavelength in the range of about 590-620 nm. In specific embodiments, the orange light may have a centroid wavelength in the 590-620 nm range. The terms "red light" or "red emission", and similar terms, may especially relate to light having a wavelength in the range of about 620-750 nm. In specific embodiments, the red light may have a centroid wavelength in the 620-750 nm range. The terms "cyan light" or "cyan emission", and similar terms, especially relate to light having a wavelength in the range of about 490-520 nm. In specific embodiments, the cyan light may have a centroid wavelength in the 490-520 nm range. The terms "amber light" or "amber emission", and similar terms, may especially relate to light having a wavelength in the range of about 585-605 nm, such as about 590-600 nm. In specific embodiments, the amber light may have a centroid wavelength in the 585-605 nm range. The phrase "light having one or more wavelengths in a wavelength range" and similar phrases may especially indicate that the indicated light (or radiation) has a spectral power distribution with at least intensity or intensities at these one or more wavelengths in the indicate wavelength range. For instance, a blue emitting solid state light source will have a spectral power distribution with intensities at one or more wavelengths in the 440-495 nm wavelength range.

The green-yellow wavelength range is defined as the 490-590 nm wavelength range. Note that the luminescent material providing luminescent material light having spectral power in the green-yellow wavelength range may in embodiments only have spectral power in this 490-590 nm wavelength range (within the 380-780 wavelength range), but may in other embodiments also have spectral power within the 380-780 wavelength range at other wavelengths than within the 490-590 nm wavelength range.

Here, a luminescent material having spectral power in the 490-590 nm wavelength range may especially not have spectral power in the 490-520 nm wavelength range only, but especially at least a major part of its spectral power outside this 490-520 nm wavelength range. For instance, more than 60% of the spectral power in the 490-590 nm wavelength range may be within the 520-590 nm wavelength range.

A broad band emitting luminescent material may be chosen such that an emission band has a full width half maximum (of the luminescent material light) of at least about 40 nm, such as at least 50 nm. For instance, the luminescent material may be chosen such that an emission band of a full width half maximum of at least 60 nm, is obtained. This may e.g. be the case with trivalent cerium comprising garnet luminescent materials (as described herein). Hence, especially the luminescent material may comprise a broad band emitter. The luminescent material may also comprise a plurality of broad band emitters. Especially, when two or more luminescent materials are applied to convert at least part of the first device light and/or at least part of the second device light, at least two of the two or more luminescent materials may be configured to provide respective luminescent material light each having an emission band with full width half maximum (of the luminescent material light) of at least 40 nm, such as at least 50 nm, like in embodiments at least 60 nm.

A narrow band emitting luminescent material may show (under excitation) an emission band having a full width half maximum (of the luminescent material light) of less than about 50 nm, such as at maximum about 40 nm. Especially, at room temperature the emission band may have a full width half maximum (of the luminescent material light) of less than about 50 nm, such as at maximum about 40 nm, like in embodiments at maximum about 35 nm, though narrow(er) line emissions may also be possible.

For instance, a broad band emitting luminescent material may have a FWHM at room temperature of larger than 45 nm, and a narrow band emitting luminescent material may have a FWHM at room temperature of at maximum 40 nm.

Alternatively or (especially) additionally, the first luminescent material may comprise a broad band emitter, having spectral power in the orange-red wavelength range.

The phrase "in the orange-red wavelength range", and similar phrases, may indicate spectral power at one or more wavelengths in the orange wavelength range and/or spectral power at one or more wavelengths in the red wavelength range. The orange-red wavelength range is defined as the 590-750 nm wavelength range. Note that the luminescent material providing luminescent material light having spectral power in the orange-red wavelength range may in embodiments only have spectral power in this 590-750 nm wavelength range (within the 380-780 nm wavelength range), but may in other embodiments also have spectral power within the 380-780 nm wavelength range at other wavelengths than within the 590-750 nm wavelength range.

Likewise, the second luminescent material may comprise one or more of broad band emitter having spectral power in the green-yellow wavelength range and a broad band emitter having spectral power in the orange-red wavelength range.

Additionally, or alternatively, more especially additionally, the first luminescent material may (further) comprise a narrow band emitter, having spectral power in the red wavelength range. Yet further, additionally, or alternatively, more especially additionally, the second luminescent material may (further) comprise a narrow band emitter, having spectral power in the red wavelength range.

In specific embodiments, the first luminescent material may comprise two or more different luminescent materials, and/or the second luminescent material may comprise two or more different luminescent materials. Above, several examples of luminescent materials have been described. For instance, for broad-band green-yellow emitters, e.g. one or more cerium comprising garnets may be selected. For (additional) broad band orange-red emitters, e.g. one or more divalent europium comprising nitrides may be chosen.

In specific embodiments, the first luminescent material and the second luminescent material may both comprise luminescent materials selected from (a) the types $A_3B_5O_{12}$:Ce, wherein A may comprise one or more of Y, La, Gd, Tb and Lu, and B may comprise one or more of Al, Ga, In and Sc, having a luminescence with spectral power at one or more wavelengths in the green-yellow wavelength range, and (b) divalent europium comprising luminescent materials having a luminescence with spectral power at one or more wavelengths in the orange-red wavelength range. The spectral power distributions of the luminescent material light of the garnets for the first light generating device and the second light generating device may differ (see also above). Alternatively or additionally, (also) the spectral power distributions of the luminescent material light of the orange-red emitting luminescent materials for the first light generating device and the second light generating device may differ; however, in other embodiments they may also be essentially the same.

Especially, the light generating system may be configured to generate system light. During operation, the system light may emanate from the light generating system. In embodiments, the system light may comprise the combined light. In specific embodiments, the system light may essentially consist of the combined light. However, in other embodiments (see also below), contribution of one or more other sources of light may also be comprised by the system light.

In embodiments, contributions of the first device light and the second device light to the system light may be controlled. In this way, the MDER value of the system light may be controlled. To this end, in embodiments a control system may be applied (see also below).

In embodiments, the light generating system may comprise a LED package. Especially, the LED package may comprise a first reflector, the first light generating device, and the second light generating device. Further, especially the first reflector may be configured to reflect at least part of the first device light and the second device light. In embodiments, the first reflector may be configured to beam shape the combined light.

In (alternative) embodiments, the light generating system may comprise a first LED package and a second LED package. The first LED package may comprise a first reflector and the first light generating device. The first reflector may be configured to reflect at least part of the first device light. The second LED package may comprise a second reflector and the second light generating device. The second reflector may be configured to reflect at least part of the second device light. Especially, the first reflector may be configured to beam shape the first device light and/or the second reflector may be configured to beam shape the second device light.

The first light generating device and the second light generating device may especially be configured to provide combined light. The combined light may have a (combined) correlated color temperature of at least about 3000 K. However, it may also be desirable to provide combined light with a relatively high CCT and further device light, from a further device, with a relatively low CCT. In this way, the CCT and/or the MDER value of the system light may further be controlled. To this end, in embodiments a control system may be applied (see also below).

Further, in embodiments the light generating system may comprise (i) a first LED string comprising one or more first light generating devices, and (ii) a second LED string comprising one or more second light generating devices. In embodiments, the control system may be configured to control the first string and second string individually.

Hence, in specific embodiments the light generating system may further comprise a third light generating device configured to generate third device light. Especially, the third device light may be white light having a third correlated color temperature $T_{c3}$. In specific embodiments $1800\,K \leq T_{c3} \leq (T_{cc} - 1000\,K)$. For instance, in embodiments $1800\,K \leq T_{c3} \leq (T_{cc} - 1500\,K)$. More especially, $1800\,K \leq T_{c3} \leq 3000\,K$. For instance, $2000\,K \leq T_{c3} \leq 3000\,K$. In other embodiments, $1800\,K \leq T_{c3} \leq 2700\,K$. Yet, in embodiments the third device light may have a color rendering index of at least 75, such as at least about 80.

In embodiments, $|(CCT1-CCT3)| \geq 1000\,K$, such as $|(CCT1-CCT3)| \geq 1500\,K$ and/or $|(CCT2-CCT3)| \geq 1000\,K$, such as $|(CCT2-CCT3)| \geq 1500\,K$.

Especially, the third light generating device may be configured to generate third device light having a primary third color point $(x_{2\_3}, y_{2\_3})$ calculated using CIE 1931 2° color matching functions, and a secondary third color point $(x_{10\_3}, y_{10\_3})$ calculated using CIE 1964 10° color matching functions. The primary third color point $(x_{2\_3}, y_{2\_3})$ and the secondary third color point $(x_{10\_3}, y_{10\_3})$ may differ, like up to several SDCMs (or even more).

In specific embodiments, the third light generating device may comprise a third (solid state) light source, configured to generate third light source light, and a third luminescent material, configured to convert at least part of the third light source light into third luminescent material light. Especially, the third light source may comprise a light emitting diode, though other (solid state) light source options may also be possible. Further, the term "third luminescent material" may refer to one or more (different) luminescent materials.

Especially, the first luminescent material, the second luminescent material, and the third luminescent material may be different luminescent materials, even though in specific embodiments they may at least partly be based on luminescent materials of the same class(es). Hence, the spectral power distribution of the first luminescent material light, the second luminescent material light, and the third luminescent material light may mutually differ. Hence, the spectral power distributions of the first luminescent material light, the second luminescent material light, and the third luminescent material light will not be identical.

The spectral power distribution of the third light source light may differ from the spectral power distribution of the first light source light and/or the spectral power distribution of the second light source light. In other embodiments, however, the spectral power distribution of the third light source light and the spectral power distribution of the first light source light may substantially be the same. The third light source light may have a third dominant wavelength $\lambda_{d3}$. In embodiments, |$(\lambda_{d3}-\lambda_{d2})$|≥10 nm, such as |$(\lambda_{d3}-\lambda_{d2})$|≥15 nm. In (other) embodiments, |$(\lambda_{d3}-\lambda_{d1})$| ≤15 nm, more especially |$(\lambda_{d3}-\lambda_{d1})$|≤10 nm, though other embodiments may also be possible.

As indicated above, the light generating system may be configured to generate system light. Especially, in embodiments the system light may comprise one or more of the combined light and the third device light. In specific embodiments, the system light may consist of the combined light and the third device light.

The light generating system further may comprise a control system, configured to control the system light. In embodiments, the control system may control in dependence of one or more of an input signal of a user interface, a sensor signal (of a sensor), and a timer (see further also below). The control system may be configured to control the spectral power distribution of the system light, such as controlling relative contributions of the combined light and the third device light. In yet other embodiments, the control system may (also) be configured to control the spectral power distribution of the combined light, such as controlling relative contributions of the first device light and second light.

Further, in embodiments the light generating system may comprise (i) a first LED string comprising one or more first light generating devices and one or more second light generating devices, and (ii) a second LED string comprising one or more third light generating devices. In embodiments, the control system may be configured to control the first string and second string individually. In this way, the CCT of the system light may be controlled. Further, in yet other embodiments the light generating system may comprise (i) a first LED string comprising one or more first light generating devices, (ii) a second LED string comprising one or more second light generating devices, and (iii) a third LED string comprising one or more third light generating devices. In embodiments, the control system may be configured to control the first string, second string, and the third string individually. In this way, the MDER and/or the CCT of the system light may be controlled.

In (other) embodiments, the system may comprise a LED package. The LED package may comprise a reflector, the first light generating device, the second light generating device, and the third light generating device. The reflector may be configured to reflect at least part of the first device light, the second device light, and the third device light. The reflector may be configured to beam shape the system light.

In yet other embodiments, the system may comprise a first LED package. The first LED package may comprise a first reflector and the first light generating device. The first reflector may be configured to reflect at least part of the first device light. Further, the system may comprise a second LED package. The second LED package may comprise a second reflector and the second light generating device. The second reflector may be configured to reflect at least part of the second device light. Further, the system may comprise a third LED package. The third LED package may comprise a third reflector and the third light generating device. The third reflector may be configured to reflect at least part of the third device light. The first reflector may be configured to beam shape the first device light. The second reflector may be configured to beam shape the second device light. The third reflector may be configured to beam shape the third device light.

In specific embodiments, the light generating system may further comprise an input device selected from the group consisting of a user interface, a time device, and a sensor. Especially, the control system may be configured to control a spectral power distribution of the system light in response to a signal of the input device.

In specific embodiments, in an operational mode of the light generation system the system light has a CRI of at least 70, such as at least 80, optionally an R9 value of at least 0, more especially at least 50, and an MDER value selected from the range of at least 0.3, more especially at least 0.45.

The light generating system may be part of or may be applied in e.g. office lighting systems, household application systems, shop lighting systems, home lighting systems, accent lighting systems, spot lighting systems, theater lighting systems, fiber-optics application systems, projection systems, self-lit display systems, pixelated display systems, segmented display systems, warning sign systems, medical lighting application systems, indicator sign systems, decorative lighting systems, portable systems, automotive applications, (outdoor) road lighting systems, urban lighting systems, green house lighting systems, horticulture lighting, digital projection, or LCD backlighting. The light generating system (or luminaire) may be part of or may be applied in e.g. optical communication systems or disinfection systems.

The term "white light", and similar terms, herein, is known to the person skilled in the art. It may especially relate to light having a correlated color temperature (CCT) between about 1800 K and 20000 K, such as between 2000 and 20000 K, especially 2700-20000 K, for general lighting especially in the range of about 2000-7000 K, such as in the range of 2700 K and 6500 K. In embodiments, e.g. for backlighting purposes, or for other purposes, the correlated color temperature (CCT) may especially be in the range of about 7000 K and 20000 K. Yet further, in embodiments the correlated color temperature (CCT) is especially within about 15 SDCM from the BBL (black body locus), especially within about 10 SDCM from the BBL, even more especially within about 5 SDCM from the BBL.

The terms "visible", "visible light" or "visible emission" and similar terms refer to light having one or more wavelengths in the range of about 380-780 nm. Herein, UV may especially refer to a wavelength selected from the range of 190-380 nm, such as 200-380 nm. The terms "light" and "radiation" are herein interchangeably used, unless clear from the context that the term "light" only refers to visible light. The terms "light" and "radiation" may thus refer to UV radiation, visible light, and IR radiation. In specific embodiments, especially for lighting applications, the terms "light" and "radiation" refer to (at least) visible light.

The term "controlling" and similar terms especially refer at least to determining the behavior or supervising the running of an element. Hence, herein "controlling" and similar terms may e.g. refer to imposing behavior to the element (determining the behavior or supervising the running of an element), etc., such as e.g. measuring, displaying, actuating, opening, shifting, changing temperature, etc. Beyond that, the term "controlling" and similar terms may additionally include monitoring. Hence, the term "controlling" and similar terms may include imposing behavior on an element and also imposing behavior on an element and monitoring the element. The controlling of the element can be done with a control system, which may also be indicated as "controller". The control system and the element may thus at least temporarily, or permanently, functionally be coupled. The element may comprise the control system. In embodiments, the control system and element may not be physically coupled. Control can be done via wired and/or wireless control. The term "control system" may also refer to a plurality of different control systems, which especially are functionally coupled, and of which e.g. one control system may be a master control system and one or more others may be slave control systems. A control system may comprise or may be functionally coupled to a user interface.

The control system may also be configured to receive and execute instructions from a remote control. In embodiments, the control system may be controlled via an App on a device, such as a portable device, like a Smartphone or I-phone, a tablet, etc. The device is thus not necessarily coupled to the lighting system, but may be (temporarily) functionally coupled to the lighting system.

Hence, in embodiments the control system may (also) be configured to be controlled by an App on a remote device. In such embodiments the control system of the lighting system may be a slave control system or control in a slave mode. For instance, the lighting system may be identifiable with a code, especially a unique code for the respective lighting system. The control system of the lighting system may be configured to be controlled by an external control system which has access to the lighting system on the basis of knowledge (input by a user interface of with an optical sensor (e.g. QR code reader) of the (unique) code. The lighting system may also comprise means for communicating with other systems or devices, such as on the basis of Bluetooth, Thread, WIFI, LiFi, ZigBee, BLE or WiMAX, or another wireless technology.

The system, or apparatus, or device may execute an action in a "mode" or "operation mode" or "mode of operation" or "operational mode". The term "operational mode may also be indicated as "controlling mode". Likewise, in a method an action or stage, or step may be executed in a "mode" or "operation mode" or "mode of operation" or "operational mode". This does not exclude that the system, or apparatus, or device may also be adapted for providing another controlling mode, or a plurality of other controlling modes. Likewise, this may not exclude that before executing the mode and/or after executing the mode one or more other modes may be executed.

However, in embodiments a control system may be available, that is adapted to provide at least the controlling mode. Would other modes be available, the choice of such modes may especially be executed via a user interface, though other options, like executing a mode in dependence of a sensor signal or a (time) scheme, may also be possible. The operation mode may in embodiments also refer to a system, or apparatus, or device, which can only operate in a single operation mode (i.e. "on", without further tunability).

Hence, in embodiments, the control system may control in dependence of one or more of an input signal of a user interface, a sensor signal (of a sensor), and a timer. The term "timer" may refer to a clock and/or a predetermined time scheme.

In yet a further aspect, the invention also provides a lamp or a luminaire comprising the light generating system as defined herein. The luminaire may further comprise a housing, optical elements, louvres, etc. etc... The lamp or luminaire may further comprise a housing enclosing the light generating system. The lamp or luminaire may comprise a light window in the housing or a housing opening, through which the system light may escape from the housing. In yet a further aspect, the invention also provides a projection device comprising the light generating system as defined herein. Especially, a projection device or "projector" or "image projector" may be an optical device that projects an image (or moving images) onto a surface, such as e.g. a projection screen. The projection device may include one or more light generating systems such as described herein. Hence, in an aspect the invention also provides a light generating device selected from the group of a lamp, a luminaire, a projector device, a disinfection device, a photochemical reactor, and an optical wireless communication device, comprising the light generating system as defined herein. The light generating device may comprise a housing or a carrier, configured to house or support, one or more elements of the light generating system. For instance, in embodiments the light generating device may comprise a housing or a carrier, configured to house or support one or more of the first light generating device and the second light generating device, and the optional third light generating device.

BRIEF DESCRIPTION OF THE DRAWINGS

Embodiments of the invention will now be described, by way of example only, with reference to the accompanying schematic drawings in which corresponding reference symbols indicate corresponding parts, and in which:

Figs. 1a-1c schematically depict some aspects of the invention;
Fig. 2 shows the 2° and 10° color matching functions;
Figs. 3a-3d show some further aspects, related to possible 2° and 10° CMF effects on the color points and spectra;
Figs. 4a-4e (schematically) depict some further aspects;
Fig. 5 shows the relative melanopic (m) (i.e. $m(\lambda)$) and $V(\lambda)$ human eye sensitivity functions; and
Fig. 6 shows some possible applications.

The schematic drawings are not necessarily to scale.

DETAILED DESCRIPTION OF THE EMBODIMENTS

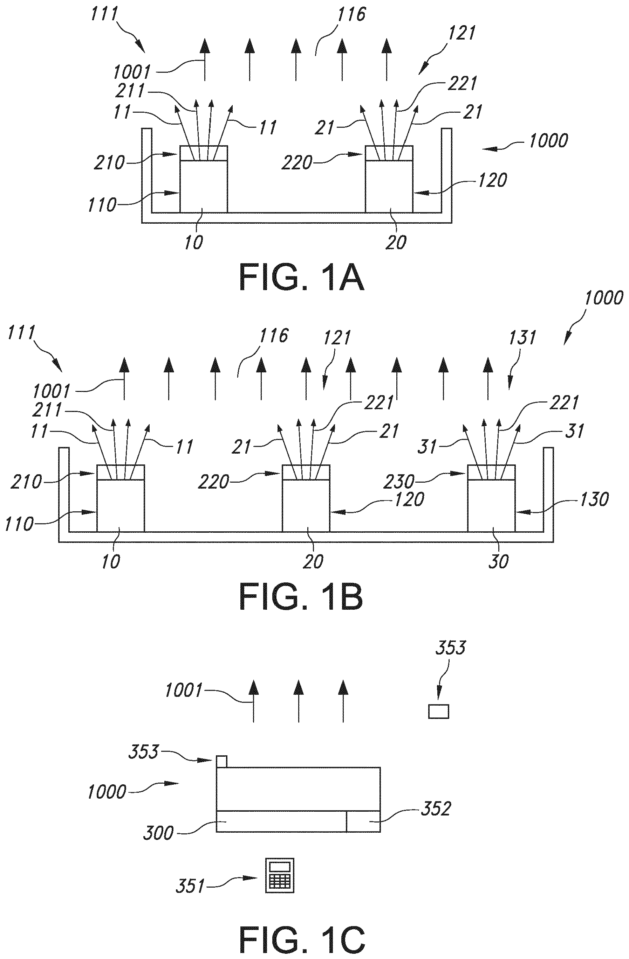

Figs. 1a-1b schematically depict embodiments of a light generating system 1000 comprising a first light generating device 110 and a second light generating device 120.

The first light generating device 110 may be configured to generate first device light 111 having a primary first color point $(x_{2\_1}, y_{2\_1})$ calculated using CIE 1931 2° color matching functions, and a secondary first color point $(x_{10\_1}, y_{10\_1})$ calculated using CIE 1964 10° color matching functions. The first light generating device 110 may comprise a first (solid state) light source 10, configured to generate first light source light 11, and a first luminescent material 210, configured to convert at least part of the first light source light 11 into first luminescent material light 211. The first light source light 11 has a first dominant wavelength $(\lambda_{d1})$ selected from the wavelength range of 440-470 nm. The first device light 111 may comprise at least part of the first light source light 11 and at least part of the first luminescent material light 211.

The second light generating device 120 may be configured to generate second device light 121 having a primary second color point $(x_{2\_2}, y_{2\_2})$ calculated using CIE 1931 2° color matching functions, and a secondary second color point $(x_{10\_2}, y_{10\_2})$ calculated using CIE 1964 10° color matching functions. The second light generating device 120 may comprise a second (solid state) light source 20, configured to generate second light source light 21, and a second luminescent material 220, configured to convert at least part of the second light source light 21 into second luminescent material light 221. The second light source light 21 has a second dominant wavelength $(\lambda_{d2})$ selected from the wavelength range of 470-500 nm. The second device light 121 may comprise at least part of the second light source light 21 and at least part of the second luminescent material light 221.

One of the first device light 111 and the second device light 121 has a relatively higher contribution in the 470-500 nm wavelength range, relative to a total spectral power distribution in the visible wavelength range, than the other one of the first device light 111 and the second device light 121. Especially, the second device light 121 has a relatively higher contribution in the 470-500 nm wavelength range, relative to a total spectral power distribution in the visible wavelength range, than the first device light 111.

Further, a difference between the primary first color point $(x_{2\_1}, y_{2\_1})$ and the primary second color point $(x_{2\_2}, y_{2\_2})$ may be within 6 SDCM.

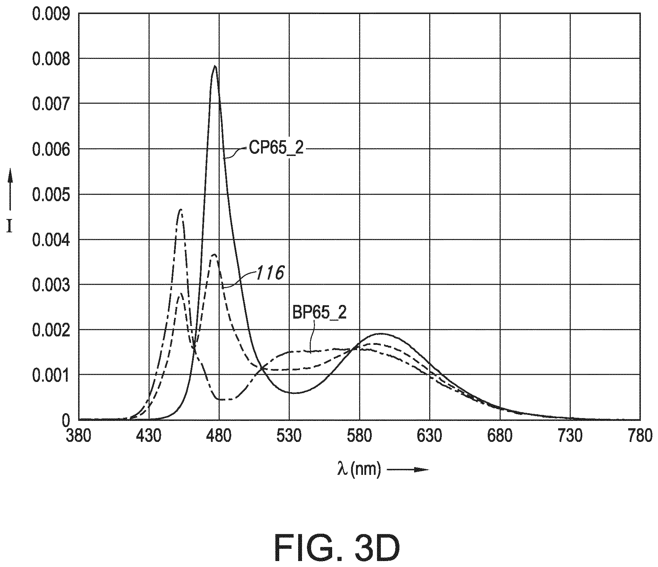

Especially, combined light 116 (see an example in Fig. 3d), comprising a combination of the first device light 111 and the second device light 121, may comprise: (i) a secondary color point $(x_{10\_sum}, y_{10\_sum})$, calculated using CIE 1964 10° color matching functions, configured within 6 SDCM from the black body locus, and (ii) a combined light correlated color temperature $(T_{cc})$ selected from the range of 3000-8000 K. The combined light correlated color temperature $(T_{cc})$ may be selected from the range of 3000-6500 K (such as in embodiments selected from the range of 3000-5000 K). In embodiments, $|(\lambda_{d1}-\lambda_{d2})| \geq 10$ nm.

Fig. 1a schematically depicts an embodiment of the system 1000 comprising the first light generating device 110 and the second light generating device 120, and Fig. 1b, schematically depicts an embodiment of the system 1000 comprising the first light generating device 110, the second light generating device 120, and the third light generating devices (see further also below).

Referring to Fig, 1c, the light generating system 1000 may further comprise an input device 350 selected from the group consisting of a user interface 351, a time device 352, and a sensor 353. The control system 300 may be configured to control a spectral power distribution of the system light 1001 in response to a signal of the input device 350.

In embodiments, the first light generating device 110 may be configured to generate the first device light 111 having at least 80% of its spectral power in the 440-500 nm wavelength range within the 440-470 nm wavelength range. Alternatively or additionally, in embodiments the second light generating device 120 may be configured to generate the second device light 121 having at least 80% of its spectral power in the 440-500 nm wavelength range within the 470-500 nm wavelength range.

In specific embodiments, a difference between the secondary first color point $(x_{10\_1},y_{10\_1})$ and the secondary second color point $(x_{10\_2},y_{10\_2})$ may be at least 5 SDCM. Further, in embodiments the secondary first color point $(x_{10\_1},y_{10\_1})$ and the secondary second color point $(x_{10\_2},y_{10\_2})$ are each configured within 20 SDCM from the black body locus.

The first luminescent material 210 may comprise two or more different luminescent materials. The second luminescent material 220 may comprise two or more different luminescent materials. The first luminescent material 210 and the second luminescent material 220 may both comprise luminescent materials selected from (a) the types $A_3B_5O_{12}$:Ce, wherein A may comprise one or more of Y, La, Gd, Tb and Lu, and B may comprise one or more of Al, Ga, In and Sc, having a luminescence with spectral power at one or more wavelengths in the green-yellow wavelength range, and (b) divalent europium comprising luminescent materials having a luminescence with spectral power at one or more wavelengths in the orange-red wavelength range.

The light generating system 1000 may be configured to generate system light 1001. Especially, the system light 1001 may comprise the combined light.

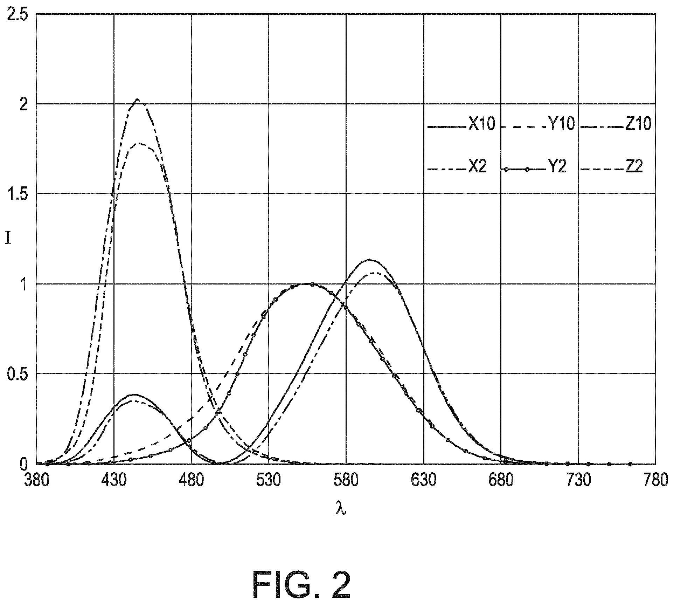

Fig. 2 provides the 2° and 10° color matching functions (see also above). As can be derived from the above, high melanopic lighting may use a cyan enhanced spectrum. To prevent a greenish appearance of the lighting in the room, the color point is herein matched to the BBL using the 10° color matching functions (CMF, large field of view). Especially for these cyan enhanced spectra a large color point difference exists between 2° (narrow field of view) and 10° (wide field of view). For 'normal' white LEDs the 2° and 10° color points are almost equal. The CMF's are displayed in Fig. 2.

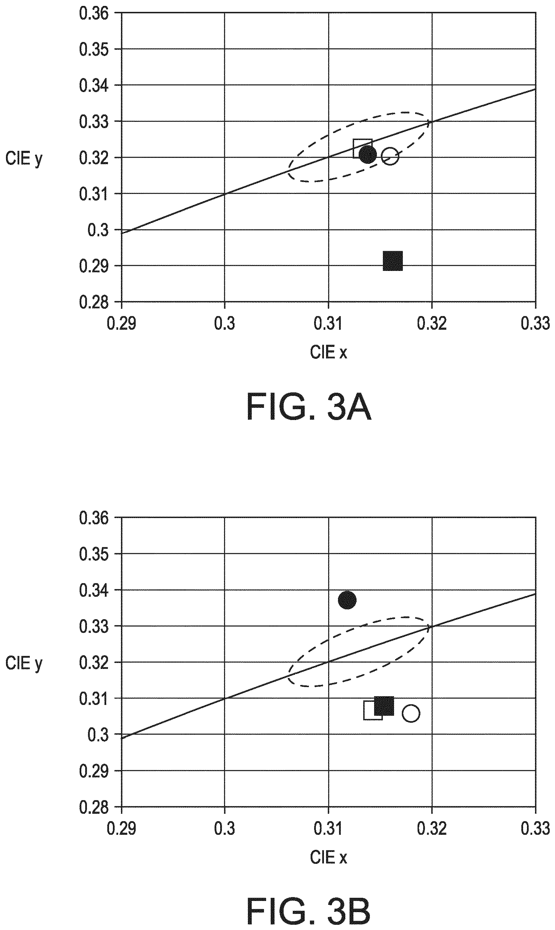

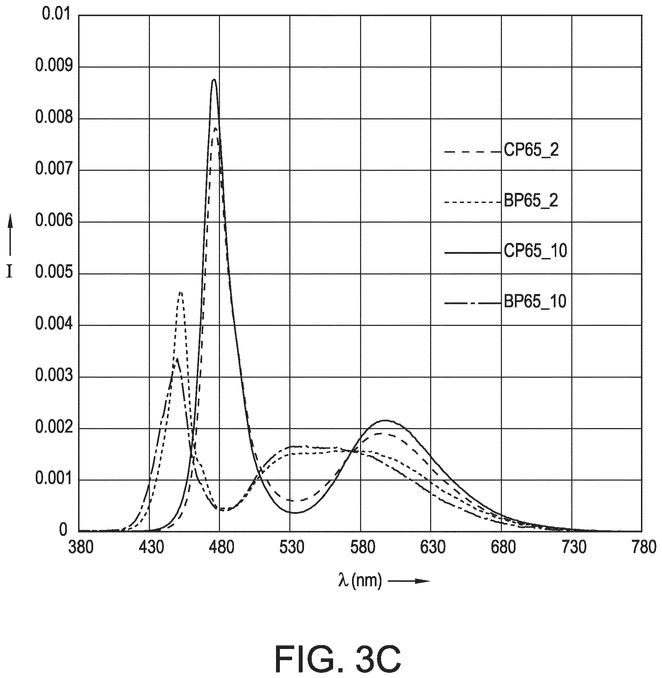

Examples of the spectra and color points can be seen in Fig. 3c and 3a. Fig. 3a shows color points. The open symbols indicate the blue pump color points and the filled symbols indicate cyan pumped color point. The squares display the 2° CMF color points and the circles display the 10° CMF color points. The dotted line indicates 5 SDCM. As can be seen from Fig. 3a, the 2° color points are substantially different while the 10° color points are substantially the same. The spectral power distributions on which these color points are based are displayed in Fig. 3c as curves CP65_10 and BP65_10, which indicate the spectral power distribution for 6500 K cyan-pumped device light and 6500 K blue-pumped device light, respectively.

Hence, in some cases, the individual color points of the LEDs can be seen (when looking at the luminaire). In these cases, the cool white LEDs have a clearly different tint: greenish versus pinkish. Luminaires for which this occurs include panels and lens luminaires, which may not be desirable or even not be acceptable.

Herein, the observable color difference between the two cool white LEDs in cyan enhanced lighting (blue versus cyan pumped) is substantially eliminated when using the invention, while good white light is observed in the environment.

Referring to Figs. 3b and 3c, LED color points are now matched in 2°, and the combined color point is matched in 10° to the BBL. Again, the squares display the 2° CMF color points and the circles display the 10° CMF color points. The dotted line indicates 5 SDCM. As can be seen, the average of 10° color points is located on the BBL, while the first and second 10° CMF color points may be located (significantly) above the BBL and (significantly) below the BBL, respectively. The spectral power distributions on which these color points are based are displayed in Fig. 3c as curves CP65_2 and BP65_2, which indicate the spectral power distribution for 6500 K cyan-pumped device light and 6500 K blue-pumped device light, respectively.

Fig. 3d schematically depict essentially the same curves CP65_2 and BP65_2, but now also with a sum curve, indicated with reference 116. The 10° color point of light 116, which is a combination of the first device light (here BP65_2) and the second device light (here CP65_2), will be essentially on the BBL (see also above).

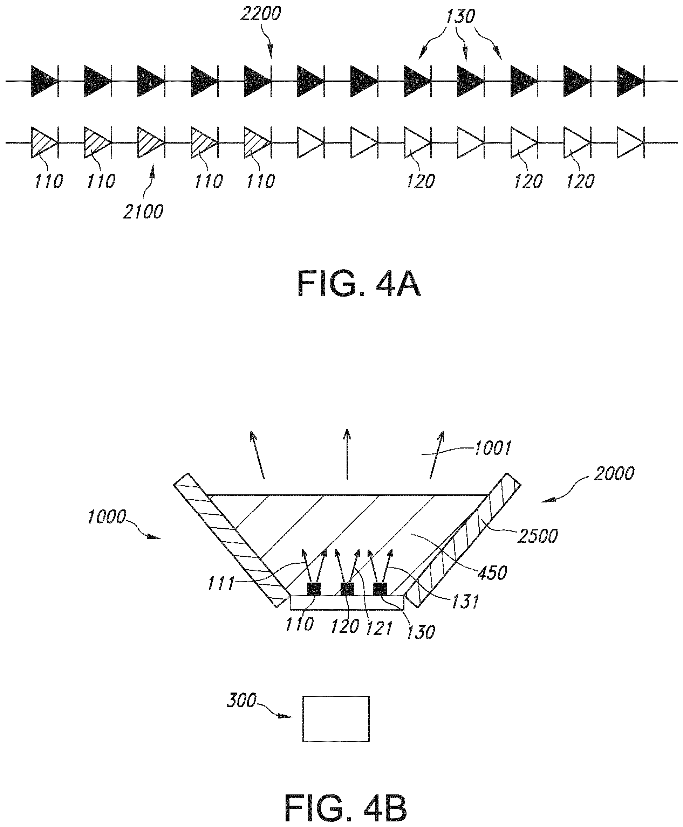

Referring to Fig. 4a, the light generating system 1000 may comprise (i) a first LED string 2100 comprising one or more first light generating devices 110 and one or more second light generating devices 120, and (ii) a second LED string 2200 comprising one or more third light generating devices 130.

In specific embodiments, see also Fig. 4a, in one of the strings there may be a number of first light generating devices 110 and a number of second light generating devices 120. A second string 2200 comprises a number of third light generating devices 130.

Referring to Fig. 4b, the system 1000 may comprise a LED package 2000. The LED package 2000 may comprise a first reflector 2500, the first light generating device 110, and the second light generating device 120. The first reflector 2500 may be configured to reflect at least part of the first device light 111 and the second device light 121. The first reflector 2500 may be configured to beam shape the combined light.

Further referring to Fig. 4b, the system 1000 may comprising a LED package 2000, wherein the LED package 2000 may comprise a reflector 2500, the first light generating device 110, the second light generating device 120, and optionally also a third light generating device 130. Especially, the reflector 2500 may be configured to reflect at least part of the first device light 111, the second device light 121, and the optional third device light 131. In specific embodiments, the reflector 2500 may (thus) be configured to beam shape the system light 1001.

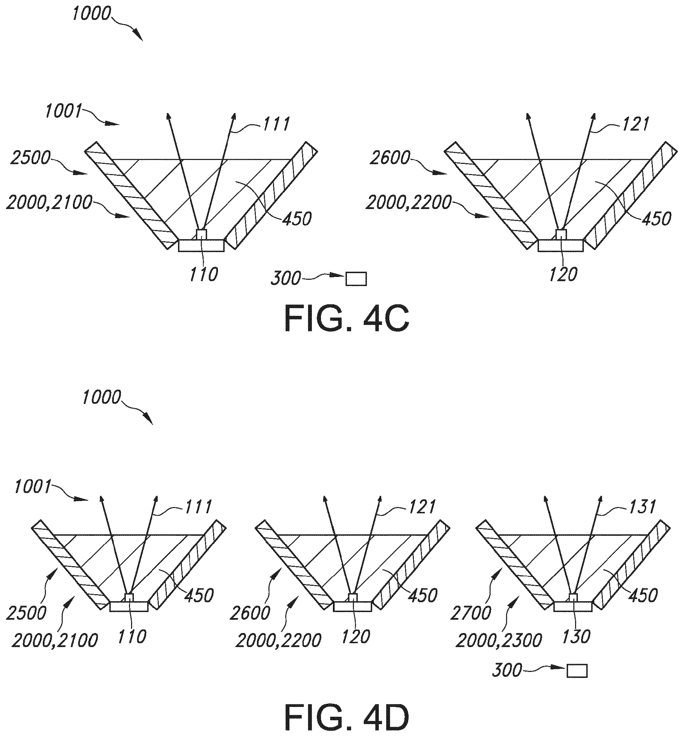

Referring to Fig. 4c, in specific embodiments, the light generating system 1000 may comprise (i) a first LED package 2100 and a second LED package 2200. The first LED package 2100 may comprise a first reflector 2500 and the first light generating device 110. The first reflector 2500 may be configured to reflect at least part of the first device light 111. The second LED package 2200 may comprise a second reflector 2600 and the second light generating device 120. The second reflector 2600 may be configured to reflect at least part of the second device light 121. The first reflector 2500 may be configured to beam shape the first device light 111. The second reflector 2600 may be configured to beam shape the second device light 121.

Referring to e.g. Fig. 4d, in specific embodiments the light generating system 1000 may further comprise a third light generating device 130 configured to generate third device light 131. The third device light 131 may be white light having a third correlated color temperature $T_{c3}$. Especially, $1800\,K \leq T_{c3} \leq (T_{cc} - 1000\,K)$. In embodiments, $1800\,K \leq T_{c3} \leq 3000\,K$. In embodiments, the third device light 131 has a color rendering index of at least 80.

Further referring to Fig. 4d, the system 1000 may comprise (i) a first LED package 2100, a second LED package 2200, and a third LED package 2300. The first LED package 2100 may comprise a first reflector 2500 and the first light generating device 110. The first reflector 2500 may be configured to reflect at least part of the first device light 111. The second LED package 2200 may comprise a second reflector 2600 and the second light generating device 120. The second reflector 2600 may be configured to reflect at least part of the second device light 121. The third LED package 2300 may comprise a third reflector 2700 and the third light generating device 130. The third reflector 2700 may be configured to reflect at least part of the third device light 131. Especially, the first reflector 2500 may be configured to beam shape the first device light 111 and/or the second reflector 2600 may be configured to beam shape the second device light 121, and/or the third reflector 2700 may be configured to beam shape the third device light 131.

Referring to Figs. 4b-4d, the light generating system 1000 may be configured to generate system light 1001. The system light 1001 may comprise one or more of the combined light 116 and the third device light 131.

The light generating system 1000 further may comprise a control system 300, configured to control the system light 1001.

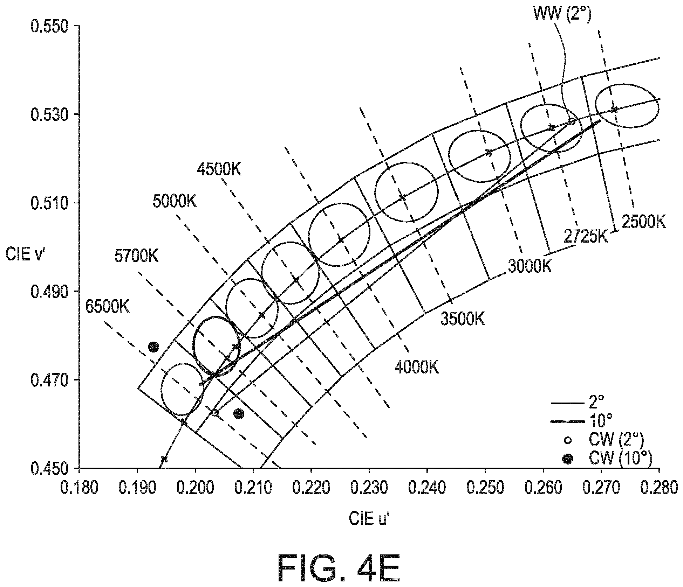

Referring to Fig. 4e, the change of 2° and 10° color points for such a tunable system can be seen, for instance a system as schematically depicted in Fig. 1b, 4a, or 4d, though other embodiments may also be tunable. Fig. 4e shows color points for a tunable system using a warm white channel, and a cool white channel consisting of a cyan-pumped cool white LED in combination with a blue pumped cool white LED. Note the relatively huge shift of the color points between 2° and 10°, especially for the cyan-pumped source. The ellipses (around the BBL) indicate 5 SDCM. The black dots at about 6500 K are the 10° color points of the first device light and the second device light. The small dot at about 2700 K is the 2° color point of the third device light. From that point, the straight line, ending at about 6500 K below the BBL, indicates the change in 2° color point (when tuning the relative current through the combined first + second light generating devices and the third light generating devices); the other line, starting at about 2600 K, and ending at about 6000 K, just above the BBL is (analogously) the change in the 10° color point.

Fig. 5 shows the relative melanopic (m) (i.e. m($\lambda$)) and photopic (V($\lambda$)) human eye sensitivity functions. The maximum sensitivity for the melanopic function is at 490 nm, the full width half maximum values are at 447 nm and 531 nm.

In an operational mode of the light generation system 1000, the system light 1001 may have one or more of a CRI of at least 80, an R9 value of at least 50, and an MDER value selected from the range of at least 0.45.

Fig. 6 schematically depicts an embodiment of a luminaire 2 comprising the light generating system 1000 as described above. Reference 301 indicates a user interface which may be functionally coupled with the control system 300 comprised by or functionally coupled to the light generating system 1000. Fig. 6 also schematically depicts an embodiment of lamp 1 comprising the light generating system 1000. Reference 3 indicates a projector device or projector system, which may be used to project images, such as at a wall, which may also comprise the light generating system 1000. Hence, Fig. 6 schematically depicts embodiments of a lighting device 1200 selected from the group of a lamp 1, a luminaire 2, a projector device 3, a disinfection device, a photochemical reactor, and an optical wireless communication device, comprising the light generating system 1000 as described herein. In embodiments, such lighting device may be a lamp 1, a luminaire 2, a projector device 3, a disinfection device, or an optical wireless communication device. Lighting device light escaping from the lighting device 1200 is indicated with reference 1201. Lighting device light 1201 may essentially consist of system light 1001, and may in specific embodiments thus be system light 1001. Reference 1300 refers to a space, such as a room. Reference 1305 refers to a floor and reference 1310 to a ceiling; reference 1307 refers to a wall.

The term "plurality" refers to two or more.

The terms "substantially" or "essentially" herein, and similar terms, will be understood by the person skilled in the art. The terms "substantially" or "essentially" may also include embodiments with "entirely", "completely", "all", etc. Hence, in embodiments the adjective substantially or essentially may also be removed. Where applicable, the term "substantially" or the term "essentially" may also relate to 90% or higher, such as 95% or higher, especially 99% or higher, even more especially 99.5% or higher, including 100%.

The term "comprise" also includes embodiments wherein the term "comprises" means "consists of".

The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices, apparatus, or systems may herein amongst others be described during operation. As will be clear to the person skilled in the art, the invention is not limited to methods of operation, or devices, apparatus, or systems in operation.

It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim.

Use of the verb "to comprise" and its conjugations does not exclude the presence of elements or steps other than those stated in a claim. Unless the context clearly requires otherwise, throughout the description and the claims, the words "comprise", "comprising", and the like are to be construed in an inclusive sense as opposed to an exclusive or exhaustive sense; that is to say, in the sense of "including, but not limited to".

The article "a" or "an" preceding an element does not exclude the presence of a plurality of such elements.

The invention may be implemented by means of hardware comprising several distinct elements, and by means of a suitably programmed computer. In a device claim, or an apparatus claim, or a system claim, enumerating several means, several of these means may be embodied by one and the same item of hardware. In yet a further aspect, the invention (thus) provides a software product, which, when running on a computer is capable of bringing about (one or more embodiments of) the method as described herein.

The invention also provides a control system that may control the device, apparatus, or system, or that may execute the herein described method or process. Yet further, the invention also provides a computer program product, when running on a computer which is functionally coupled to or comprised by the device, apparatus, or system, controls one or more controllable elements of such device, apparatus, or system.

## Claims

1. A light generating system (1000) comprising a first light generating device (110) and a second light generating device (120), wherein:

   - the first light generating device (110) is configured to generate first device light (111) having a primary first color point $(x_{2\_1}, y_{2\_1})$ calculated using CIE 1931 2° color matching functions, and a secondary first color point $(x_{10\_1}, y_{10\_1})$ calculated using CIE 1964 10° color matching functions; wherein the first light generating device (110) comprises a first solid state light source (10), configured to generate first light source light (11), wherein the first light generating device (110) comprises a first luminescent material (210), configured to convert at least part of the first light source light (11) into first luminescent material light (211), wherein the first light source light (11) has a first dominant wavelength $(\lambda_{d1})$ selected from the wavelength range of 440-470 nm, and wherein the first device light (111) comprises at least part of the first light source light (11) and at least part of the first luminescent material light (211);
   - the second light generating device (120) is configured to generate second device light (121) having a primary second color point $(x_{2\_2}, y_{2\_2})$ calculated using CIE 1931 2° color matching functions, and a secondary second color point $(x_{10\_2}, y_{10\_2})$ calculated using CIE 1964 10° color matching functions; wherein the second light generating device (120) comprises a second solid state light source (20), configured to generate second light source light (21), and a second luminescent material (220), configured to convert at least part of the second light source light (21) into second luminescent material light (221), wherein the second light source light (21) has a second dominant wavelength $(\lambda_{d2})$ selected from the wavelength range of 470-500 nm, and wherein the second device light (121) comprises at least part of the second light source light (21) and at least part of the second luminescent material light (221);
   - the first light generating device (110) is configured to generate the first device light (111) in the 440-500 nm wavelength range, having at least 80% of its spectral power within the 440-470 nm wavelength range and the second light generating device (120) is configured to generate the second device light (121) in the 440-500 nm wavelength range, having at least 80% of its spectral power within the 470-500 nm wavelength range, wherein one of the first device light (111) and the second device light (121) has a relatively higher contribution in the 470-500 nm wavelength range, relative to a total spectral power distribution in the visible wavelength range, than the other one of the first device light (111) and the second device light (121);
- the first dominant wavelength ($\lambda_{d1}$) and the second dominant wavelength ($\lambda_{d2}$) differ by at least 10 nm;
- a difference between the primary first color point ($x_{2\_1}$,$y_{2\_1}$) and the primary second color point ($x_{2\_2}$,$y_{2\_2}$) is within 6 SDCM (standard deviation of color matching);
- combined light, comprising a combination of the first device light (111) and the second device light (121), comprises: (i) a secondary color point ($x_{10\_sum}$,$y_{10\_sum}$), calculated using CIE 1964 10° color matching functions, configured within 6 SDCM from the black body locus, and (ii) a combined light correlated color temperature, $T_{cc}$, selected from the range of 3000-8000 K.

2. The light generating system (1000) according to claim 1, wherein the secondary first color point ($x_{10\_1}$,$y_{10\_1}$) calculated using CIE 1964 10° color matching functions is above the black body locus, wherein the secondary second color point ($x_{10\_2}$,$y_{10\_2}$) calculated using CIE 1964 10° color matching functions is below the black body locus, and wherein the secondary color point ($x_{10\_sum}$,$y_{10\_sum}$) calculated using CIE 1964 10° color matching functions is configured within 6 SDCM from the black body locus.

3. The light generating system (1000) according to any one of the preceding claims, wherein a difference between the secondary first color point ($x_{10\_1}$,$y_{10\_1}$) and the secondary second color point ($x_{10\_2}$,$y_{10\_2}$) is at least 5 SDCM.

4. The light generating system (1000) according to any one of the preceding claims, wherein the secondary first color point ($x_{10\_1}$,$y_{10\_1}$) and the secondary second color point ($x_{10\_2}$,$y_{10\_2}$) are each configured within 20 SDCM from the black body locus.

5. The light generating system (1000) according to any one of the preceding claims, wherein the combined light correlated color temperature, $T_{cc}$, is selected from the range of 3000-6500 K.

6. The light generating system (1000) according to any one of the preceding claims, wherein the combined light correlated color temperature, $T_{cc}$, is selected from the range of 3000-5000 K.

7. The light generating system (1000) according to any one of the preceding claims, wherein the first luminescent material (210) comprises two or more different luminescent materials, and wherein the second luminescent material (220) comprises two or more different luminescent materials; wherein the first luminescent material (210) and the second luminescent material (220) both comprise luminescent materials selected from (a) the types $A_3B_5O_{12}$:Ce, wherein A comprises one or more of Y, La, Gd, Tb and Lu, and wherein B comprises one or more of Al, Ga, In and Sc, having a luminescence with spectral power at one or more wavelengths in the green-yellow wavelength range, and (b) divalent europium comprising luminescent materials having a luminescence with spectral power at one or more wavelengths in the orange-red wavelength range.

8. The light generating system (1000) according to any one of the preceding claims, comprising (i) a first LED package (2100), wherein the first LED package (2100) comprises a first reflector (2500) and the first light generating device (110), wherein the first reflector (2500) is configured to reflect at least part of the first device light (111), and (b) a second LED package (2200), wherein the second LED package (2200) comprises a second reflector (2600) and the second light generating device (120), wherein the second reflector (2600) is configured to reflect at least part of the second device light (121).

9. The light generating system (1000) according to any one of the preceding claims, further comprising a third light generating device (130) configured to generate third device light (131), wherein the third device light (131) is white light having a third correlated color temperature, $T_{c3}$, wherein 1800 K $\leq$ $T_{c3}$ $\leq$ ($T_{cc}$ - 1000 K).

10. The light generating system (1000) according to claim 9, wherein 1800 K $\leq$ $T_{c3}$ $\leq$ 3000 K.

11. The light generating system (1000) according to any one of the preceding claims 9-10, wherein the third device light (131) has a color rendering index of at least 80.

12. The light generating system (1000) according to any one of the preceding claims 9-11, wherein the light generating system (1000) is configured to generate system light (1001), wherein the system light (1001) comprises one or more of the combined light and the third device light (131), wherein the light generating system (1000) further comprises a control system (300), configured to control the system light (1001).

13. The light generating system (1000) according to any one of the preceding claims 9-12, comprising (i) a first LED string (2100) comprising one or more first light generating devices (110) and one or more second light generating devices (120), and (ii) a second LED string (2200) comprising one or more third light generating devices (130).

14. The light generating system (1000) according to claims 12-13, further comprising an input device (350) selected from the group consisting of a user interface (351), a time device (352), and a sensor (353), wherein the control system (300) is configured to control a spectral power distribution of the system light (1001) in response to a signal of the input device (350).

15. A lighting device (1200) selected from the group of a lamp (1), a luminaire (2), a projector device (3), a disinfection device, a photochemical reactor, and an optical wireless communication device, comprising the light generating system (1000) according to any one of the preceding claims.

**Patentansprüche**

1. Lichterzeugungssystem (1000), umfassend eine erste Lichterzeugungsvorrichtung (110) und eine zweite Lichterzeugungsvorrichtung (120), wobei:

- die erste Lichterzeugungsvorrichtung (110) konfiguriert ist, um ein erstes Vorrichtungslicht (111) zu erzeugen, das einen primären ersten Farbpunkt ($x_{2\_1}$,$y_{2\_1}$), berechnet unter Verwendung von CIE-1931-2°-Farbwertfunktionen, und einen sekundären ersten Farbpunkt ($x_{10\_1}$,$y_{10\_1}$), berechnet unter Verwendung von CIE-1964-10°-Farbwertfunktionen, aufweist; wobei die erste Lichterzeugungsvorrichtung (110) eine erste Festkörperlichtquelle (10) umfasst, die konfiguriert ist, um ein erstes Lichtquellenlicht (11) zu erzeugen, wobei die erste Lichterzeugungsvorrichtung (110) einen ersten Leuchtstoff (210) umfasst, der konfiguriert ist, um mindestens einen Teil des ersten Lichtquellenlichts (11) in ein erstes Leuchtstofflicht (211) umzuwandeln, wobei das erste Lichtquellenlicht (11) eine erste farbtongleiche Wellenlänge ($\lambda_{d1}$) aufweist, die aus dem Wellenlängenbereich von 440 bis 470 nm ausgewählt ist, und wobei das erste Vorrichtungslicht (111) mindestens einen Teil des ersten Lichtquellenlichts (11) und mindestens einen Teil des ersten Leuchtstofflichts (211) umfasst;
- die zweite Lichterzeugungsvorrichtung (120) konfiguriert ist, um ein zweites Vorrichtungslicht (121) zu erzeugen, das einen primären zweiten Farbpunkt ($x_{2\_2}$,$y_{2\_2}$), berechnet unter Verwendung von CIE-1931-2°-Farbwertfunktionen, und einen sekundären zweiten Farbpunkt ($x_{10\_2}$,$y_{10\_2}$), berechnet unter Verwendung von CIE-1964-10°-Farbwertfunktionen, aufweist; wobei die zweite Lichterzeugungsvorrichtung (120) eine zweite Festkörperlichtquelle (20), die konfiguriert ist, um ein zweites Lichtquellenlicht (21) zu erzeugen, und einen zweiten Leuchtstoff (220), der konfiguriert ist, um mindestens einen Teil des zweiten Lichtquellenlichts (21) in ein zweites Leuchtstofflicht (221) umzuwandeln, umfasst, wobei das zweite Lichtquellenlicht (21) eine zweite farbtongleiche Wellenlänge ($\lambda_{d2}$) aufweist, die aus dem Wellenlängenbereich von 470 bis 500 nm ausgewählt ist, und wobei das zweite Vorrichtungslicht (121) mindestens einen Teil des zweiten Lichtquellenlichts (21) und mindestens einen Teil des zweiten Leuchtstofflichts (221) umfasst;
- die erste Lichterzeugungsvorrichtung (110) konfiguriert ist, um das erste Vorrichtungslicht (111) im Wellenlängenbereich von 440 bis 500 nm zu erzeugen, das mindestens 80 % seiner spektralen Leistung innerhalb des Wellenlängenbereichs von 440 bis 470 nm aufweist, und die zweite Lichterzeugungsvorrichtung (120) konfiguriert ist, um das zweite Vorrichtungslicht (121) im Wellenlängenbereich von 440 bis 500 nm zu erzeugen, das mindestens 80 % seiner spektralen Leistung innerhalb des Wellenlängenbereichs von 470 bis 500 nm aufweist, wobei eines von dem ersten Vorrichtungslicht (111) und dem zweiten Vorrichtungslicht (121) einen relativ höheren Beitrag im Wellenlängenbereich von 470 bis 500 nm, relativ zu einer gesamten spektralen Leistungsverteilung im sichtbaren Wellenlängenbereich, als das andere von dem ersten Vorrichtungslicht (111) und dem zweiten Vorrichtungslicht (121) aufweist;
- die erste farbtongleiche Wellenlänge ($\lambda_{d1}$) und die zweite farbtongleiche Wellenlänge ($\lambda_{d2}$) sich um mindestens 10 nm unterscheiden;
- eine Differenz zwischen dem primären ersten Farbpunkt ($x_{2\_1}$,$y_{2\_1}$) und dem primären zweiten Farbpunkt ($x_{2\_2}$,$y_{2\_2}$) innerhalb von 6 SDCM (Standardabweichung des Farbabgleichs) liegt;
- kombiniertes Licht, umfassend eine Kombination des ersten Vorrichtungslichts (111) und des zweiten Vorrichtungslichts (121), umfasst: (i) einen sekundäre Farbpunkt ($x_{10\_Summe}$,$y_{10\_Summe}$), berechnet unter Verwendung von CIE-1964-10°-Farbwertfunktionen, konfiguriert innerhalb von 6 SDCM von der Schwarzkörperkurve, und (ii) eine korrelierte Farbtemperatur des kombinierten Lichts, $T_{cc}$, die aus dem Bereich von 3000 bis 8000 K ausgewählt ist.

2. Lichterzeugungssystem (1000) nach Anspruch 1, wobei der unter Verwendung von CIE-1964-10°-Farbwertfunktionen berechnete sekundäre erste Farbpunkt ($x_{10\_1}$,$y_{10\_1}$) oberhalb der Schwarzkörperkurve liegt, wobei der unter Verwendung von CIE-1964-10°-Farbwertfunktionen berechnete sekundäre zweite Farbpunkt ($x_{10\_2}$,$y_{10\_2}$) unterhalb der Schwarzkörperkurve liegt und wobei der unter Verwendung von CIE-1964-10°-Farbwertfunktionen berechnete sekundäre Farbpunkt ($x_{10\_Summe}$,$y_{10\_Summe}$) innerhalb von 6 SDCM von der Schwarzkörperkurve konfiguriert ist.

3. Lichterzeugungssystem (1000) nach einem der vorstehenden Ansprüche, wobei eine Differenz zwischen dem sekundären ersten Farbpunkt ($x_{10\_1}$,$y_{10\_1}$) und dem sekundären zweiten Farbpunkt ($x_{10\_2}$,$y_{10\_2}$) mindestens 5 SDCM beträgt.

4. Lichterzeugungssystem (1000) nach einem der vorstehenden Ansprüche, wobei der sekundäre erste Farbpunkt ($x_{10\_1}$,$y_{10\_1}$) und der sekundäre zweite Farbpunkt ($x_{10\_2}$,$y_{10\_2}$) jeweils innerhalb von 20 SDCM von der Schwarzkörperkurve konfiguriert sind.

5. Lichterzeugungssystem (1000) nach einem der vorstehenden Ansprüche, wobei die korrelierte Farbtemperatur des kombinierten Lichts, $T_{cc}$, aus dem Bereich von 3000 bis 6500 K ausgewählt ist.

6. Lichterzeugungssystem (1000) nach einem der vorstehenden Ansprüche, wobei die korrelierte Farbtemperatur des kombinierten Lichts, $T_{cc}$, aus dem Bereich von 3000 bis 5000 K ausgewählt ist.

7. Lichterzeugungssystem (1000) nach einem der vorstehenden Ansprüche, wobei der erste Leuchtstoff (210) zwei oder mehrere unterschiedliche Leuchtstoffe umfasst und wobei der zweite Leuchtstoff (220) zwei oder mehrere unterschiedliche Leuchtstoffe umfasst; wobei der erste Leuchtstoff (210) und der zweite Leuchtstoff (220) beide Leuchtstoffe umfassen, die ausgewählt sind aus (a) den Typen $A_3B_5O_{12}$:Ce, wobei A eines oder mehrere von Y, La, Gd, Tb und Lu umfasst und wobei B eines oder mehrere von Al, Ga, In und Sc umfasst, die eine Lumineszenz mit spektraler Leistung bei einer oder mehreren Wellenlänge(n) im grün-gelben Wellenlängenbereich aufweisen, und (b) zweiwertiges Europium umfassenden Leuchtstoffen, die eine Lumineszenz mit spektraler Leistung bei einer oder mehreren Wellenlänge(n) im orange-roten Wellenlängenbereich aufweisen.

8. Lichterzeugungssystem (1000) nach einem der vorstehenden Ansprüche, umfassend (i) ein erstes LED-Package (2100), wobei das erste LED-Package (2100) einen ersten Reflektor (2500) und die erste Lichterzeugungsvorrichtung (110) umfasst, wobei der erste Reflektor (2500) konfiguriert ist, um mindestens einen Teil des ersten Vorrichtungslichts (111) zu reflektieren, und (b) ein zweites LED-Package (2200), wobei das zweite LED-Package (2200) einen zweiten Reflektor (2600) und die zweite Lichterzeugungsvorrichtung (120) umfasst, wobei der zweite Reflektor (2600) konfiguriert ist, um mindestens einen Teil des zweiten Vorrichtungslichts (121) zu reflektieren.

9. Lichterzeugungssystem (1000) nach einem der vorstehenden Ansprüche, ferner umfassend eine dritte Lichterzeugungsvorrichtung (130), die konfiguriert ist, um drittes Vorrichtungslicht (131) zu erzeugen, wobei das dritte Vorrichtungslicht (131) weißes Licht ist, das eine dritte korrelierten Farbtemperatur, $T_{c3}$, aufweist, wobei $1800\,K \le T_{c3} \le (T_{cc} - 1000\,K)$ ist.

10. Lichterzeugungssystem (1000) nach Anspruch 9, wobei $1800\,K \le T_{c3} \le 3000\,K$ ist.

11. Lichterzeugungssystem (1000) nach einem der vorstehenden Ansprüche 9 bis 10, wobei das dritte Vorrichtungslicht (131) einen Farbwiedergabeindex von mindestens 80 aufweist.

12. Lichterzeugungssystem (1000) nach einem der vorstehenden Ansprüche 9 bis 11, wobei das Lichterzeugungssystem (1000) konfiguriert ist, um ein Systemlicht (1001) zu erzeugen, wobei das Systemlicht (1001) eines oder mehrere von dem kombinierten Licht und dem dritten Vorrichtungslicht (131) umfasst, wobei das Lichterzeugungssystem (1000) ferner ein Steuersystem (300) umfasst, das konfiguriert ist, um das Systemlicht (1001) zu steuern.

13. Lichterzeugungssystem (1000) nach einem der vorstehenden Ansprüche 9 bis 12, umfassend (i) einen ersten LED-Strang (2100), umfassend eine oder mehrere erste Lichterzeugungsvorrichtung(en) (110) und eine oder mehrere zweite Lichterzeugungsvorrichtung(en) (120), und (ii) einen zweiten LED-Strang (2200), umfassend eine oder mehrere dritte Lichterzeugungsvorrichtung(en) (130).

**14.** Lichterzeugungssystem (1000) nach Anspruch 12 bis 13, ferner umfassend eine Eingabevorrichtung (350), die aus der Gruppe ausgewählt ist, bestehend aus einer Benutzerschnittstelle (351), einer Zeitvorrichtung (352) und einem Sensor (353), wobei das Steuersystem (300) konfiguriert ist, um eine spektrale Leistungsverteilung des Systemlichts (1001) als Reaktion auf ein Signal der Eingabevorrichtung (350) zu steuern.

**15.** Beleuchtungsvorrichtung (1200), die aus der Gruppe einer Lampe (1), einer Leuchte (2), einer Projektorvorrichtung (3), einer Desinfektionsvorrichtung, einem photochemischen Reaktor und einer optischen drahtlosen Kommunikationsvorrichtung ausgewählt ist, umfassend das Lichterzeugungssystem (1000) nach einem der vorstehenden Ansprüche.

**Revendications**

**1.** Système générateur de lumière (1000) comprenant un premier dispositif générateur de lumière (110) et un deuxième dispositif générateur de lumière (120), dans lequel :

- le premier dispositif générateur de lumière (110) est configuré pour générer de la lumière de premier dispositif (111) ayant un premier point de couleur primaire $(x_{2\_1},y_{2\_1})$ calculé à l'aide de fonctions de correspondance de couleurs à 2° de CIE 1931, et un premier point de couleur secondaire $(x_{10\_1},y_{10\_1})$ calculé à l'aide de fonctions de correspondance de couleurs à 10° de CIE 1964 ; dans lequel le premier dispositif générateur de lumière (110) comprend une première source de lumière à semi-conducteurs (10), configurée pour générer de la lumière de première source de lumière (11), dans lequel le premier dispositif générateur de lumière (110) comprend un premier matériau luminescent (210), configuré pour convertir au moins une partie de la lumière de première source de lumière (11) en lumière de premier matériau luminescent (211), dans lequel la lumière de première source de lumière (11) a une première longueur d'onde dominante $(\lambda_{d1})$ sélectionnée parmi la plage de longueur d'onde de 440 à 470 nm, et dans lequel la lumière de premier dispositif (111) comprend au moins une partie de la lumière de première source de lumière (11) et au moins une partie de la lumière de premier matériau luminescent (211) ;
- le deuxième dispositif générateur de lumière (120) est configuré pour générer de la lumière de deuxième dispositif (121) ayant un second point de couleur primaire $(x_{2\_2},y_{2\_2})$ calculé à l'aide de fonctions de correspondance de couleurs à 2° de CIE 1931, et un second point de couleur secondaire $(x_{10\_2},y_{10\_2})$ calculé à l'aide de fonctions de correspondance de couleurs à 10° de CIE 1964 ; dans lequel le deuxième dispositif générateur de lumière (120) comprend une seconde source de lumière à semi-conducteurs (20), configurée pour générer de la lumière de seconde source de lumière (21), et un second matériau luminescent (220), configuré pour convertir au moins une partie de la lumière de seconde source de lumière (21) en lumière de second matériau luminescent (221), dans lequel la lumière de seconde source de lumière (21) a une seconde longueur d'onde dominante $(\lambda_{d2})$ sélectionnée parmi la plage de longueur d'onde de 470 à 500 nm, et dans lequel la lumière de deuxième dispositif (121) comprend au moins une partie de la lumière de seconde source de lumière (21) et au moins une partie de la lumière de second matériau luminescent (221) ;
- le premier dispositif générateur de lumière (110) est configuré pour générer la lumière de premier dispositif (111) dans la plage de longueur d'onde de 440 à 500 nm, ayant au moins 80 % de sa puissance spectrale au sein de la plage de longueur d'onde de 440 à 470 nm et le deuxième dispositif générateur de lumière (120) est configuré pour générer la lumière de deuxième dispositif (121) dans la plage de longueur d'onde de 440 à 500 nm, ayant au moins 80 % de sa puissance spectrale au sein de la plage de longueur d'onde de 470 à 500 nm, dans lequel l'une de la lumière de premier dispositif (111) et de la lumière de deuxième dispositif (121) a une contribution relativement plus élevée dans la plage de longueur d'onde de 470 à 500 nm, par rapport à une distribution de puissance spectrale totale dans la plage de longueur d'onde visible, que l'autre de la lumière de premier dispositif (111) et de la lumière de deuxième dispositif (121) ;
- la première longueur d'onde dominante $(\lambda_{d1})$ et la seconde longueur d'onde dominante $(\lambda_{d2})$ diffèrent d'au moins 10 nm ;
- une différence entre le premier point de couleur primaire $(x_{2\_1},y_{2\_1})$ et le second point de couleur primaire $(x_{2\_2},y_{2\_2})$ est à plus ou moins 6 SDCM (écart-type de correspondance de couleur) ;
- la lumière combinée, comprenant une combinaison de la lumière de premier dispositif (111) et de la lumière de deuxième dispositif (121), comprend : (i) un point de couleur secondaire $(x_{10\_sum},y_{10\_sum})$, calculé à l'aide de fonctions de correspondance de couleurs à 10° de CIE 1964, configuré à plus ou moins de 6 SDCM par rapport au lieu de corps noir, et (ii) une température de couleur corrélée de lumière combinée, $T_{cc}$, sélectionnée dans la plage de 3000 à 8000 K.

**2.** Système générateur de lumière (1000) selon la revendication 1, dans lequel le premier point de couleur secondaire $(x_{10\_1}, y_{10\_1})$ calculé à l'aide de fonctions de correspondance de couleurs à 10° de CIE 1964 est au-dessus du lieu de corps noir, dans lequel le second point de couleur secondaire $(x_{10\_2}, y_{10\_2})$ calculé à l'aide de fonctions de correspondance de couleurs à 10° de CIE 1964 est en dessous du lieu de corps noir, et dans lequel le point de couleur secondaire $(x_{10\_sum}, y_{10\_sum})$ calculé à l'aide de fonctions de correspondance de couleurs à 10° de CIE 1964 est configuré à plus ou moins de 6 SDCM par rapport au lieu de corps noir.

**3.** Système générateur de lumière (1000) selon l'une quelconque des revendications précédentes, dans lequel une différence entre le premier point de couleur secondaire $(x_{10\_1}, y_{10\_1})$ et le second point de couleur secondaire $(x_{10\_2}, y_{10\_2})$ est d'au moins 5 SDCM.

**4.** Système générateur de lumière (1000) selon l'une quelconque des revendications précédentes, dans lequel le premier point de couleur secondaire $(x_{10\_1}, y_{10\_1})$ et le second point de couleur secondaire $(x_{10\_2}, y_{10\_2})$ sont configurés chacun à plus ou moins 20 SDCM par rapport au lieu de corps noir.

**5.** Système générateur de lumière (1000) selon l'une quelconque des revendications précédentes, dans lequel la température de couleur corrélée de lumière combinée, $T_{cc}$, est sélectionnée dans la plage de 3000 à 6500 K.

**6.** Système générateur de lumière (1000) selon l'une quelconque des revendications précédentes, dans lequel la température de couleur corrélée de lumière combinée, $T_{cc}$, est sélectionnée dans la plage de 3000 à 5000 K.

**7.** Système générateur de lumière (1000) selon l'une quelconque des revendications précédentes, dans lequel le premier matériau luminescent (210) comprend deux matériaux luminescents différents ou plus, et dans lequel le second matériau luminescent (220) comprend deux matériaux luminescents différents ou plus ; dans lequel le premier matériau luminescent (210) et le second matériau luminescent (220) comprennent l'un et l'autre des matériaux luminescents sélectionnés parmi (a) les types $A_3B_5O_{12}$:Ce, dans lequel A comprend un ou plusieurs parmi Y, La, Gd, Tb et Lu, et dans lequel B comprend un ou plusieurs parmi Al, Ga, In et Sc, ayant une luminescence avec une puissance spectrale au niveau d'une ou plusieurs longueurs d'onde dans la plage de longueur d'onde vert-jaune, et (b) de l'europium divalent comprenant des matériaux luminescents ayant une luminescence avec une puissance spectrale au niveau d'une ou plusieurs longueurs d'onde dans la plage de longueur d'onde orange-rouge.

**8.** Système générateur de lumière (1000) selon l'une quelconque des revendications précédentes, comprenant (i) un premier boîtier de DEL (2100), dans lequel le premier boîtier de DEL (2100) comprend un premier réflecteur (2500) et le premier dispositif générateur de lumière (110), dans lequel le premier réflecteur (2500) est configuré pour réfléchir au moins une partie de la lumière de premier dispositif (111), et (b) un second boîtier de DEL (2200), dans lequel le second boîtier de DEL (2200) comprend un second réflecteur (2600) et le deuxième dispositif générateur de lumière (120), dans lequel le second réflecteur (2600) est configuré pour réfléchir au moins une partie de la lumière de deuxième dispositif (121).

**9.** Système générateur de lumière (1000) selon l'une quelconque des revendications précédentes, comprenant en outre un troisième dispositif générateur de lumière (130) configuré pour générer de la lumière de troisième dispositif (131), dans lequel la lumière de troisième dispositif (131) est de la lumière blanche ayant une troisième température de couleur corrélée, $T_{c3}$, dans lequel 1800 K ≤ $T_{c3}$ ≤ ($T_{cc}$ - 1000 K).

**10.** Système générateur de lumière (1000) selon la revendication 9, dans lequel 1800 K ≤ $T_{c3}$ ≤ 3000 K.

**11.** Système générateur de lumière (1000) selon l'une quelconque des revendications 9 à 10 précédentes, dans lequel la lumière de troisième dispositif (131) a un indice de restitution de couleurs d'au moins 80.

**12.** Système générateur de lumière (1000) selon l'une quelconque des revendications 9 à 11 précédentes, dans lequel le système générateur de lumière (1000) est configuré pour générer de la lumière de système (1001), dans lequel la lumière de système (1001) comprend une ou plusieurs de la lumière combinée et de la lumière de troisième dispositif (131), dans lequel le système générateur de lumière (1000) comprend en outre un système de commande (300), configuré pour commander la lumière de système (1001).

**13.** Système générateur de lumière (1000) selon l'une quelconque des revendications 9 à 12 précédentes, comprenant (i) un premier chapelet de DEL (2100) comprenant un ou plusieurs premiers dispositifs générateurs de lumière (110) et un ou plusieurs deuxièmes dispositifs générateurs de lumière (120), et (ii) un second chapelet de DEL (2200) comprenant un ou plusieurs troisièmes dispositifs générateurs de lumière (130).

14. Système générateur de lumière (1000) selon les revendications 12 à 13, comprenant en outre un dispositif d'entrée (350) sélectionné dans le groupe constitué d'une interface utilisateur (351), d'un dispositif temporel (352) et d'un capteur (353), dans lequel le système de commande (300) est configuré pour commander une distribution de puissance spectrale de la lumière de système (1001) en réponse à un signal du dispositif d'entrée (350).

15. Dispositif d'éclairage (1200) sélectionné dans le groupe d'une lampe (1), d'un luminaire (2), d'un dispositif projecteur (3), d'un dispositif de désinfection, d'un réacteur photochimique et d'un dispositif de communication optique sans fil, comprenant le système générateur de lumière (1000) selon l'une quelconque des revendications précédentes.

**FIG. 1A**

**FIG. 1B**

**FIG. 1C**

FIG. 2

FIG. 3A

FIG. 3B

FIG. 3C

FIG. 3D

2200

130

2100

110 110 110 110 120 120 120

## FIG. 4A

1001

2000

1000

2500

450

111

131

110 120 121 130

300

## FIG. 4B

1000

1001

2500

2000,2100

111

450

110

300

FIG. 4C

2600

2000,2200

121

450

120

1000

1001

2500

2000,2100 110

111

450

2600

2000,2200 120

121

450

2700

2000,2300 130

131

450

300

FIG. 4D

FIG. 4E

FIG. 5

FIG. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9039746 B
- US 20180056027 A1
- EP 3149108 A

**Non-patent literature cited in the description**

- **R.J. LUCAS et al.** Measuring and using light in the melanopsin age. *Trends in Neurosciences*, January 2014, vol. 37 (1), 1-9, http://www.sciencedirect.com/science/article/pii/S0166223613001975
- *CIE TN 003:2015 : Report on the First International Workshop on Circadian and Neurophysiological Photometry, 2013*, http://cie.co.at/index.php?i_ca_id=978